(19) Europäisches Patentamt / European Patent Office / Office européen des brevets

(11) **EP 2 945 601 B1**

(12) **EUROPEAN PATENT SPECIFICATION**

(45) Date of publication and mention of the grant of the patent:
**22.08.2018 Bulletin 2018/34**

(21) Application number: **14700911.2**

(22) Date of filing: **20.01.2014**

(51) Int Cl.:
***A61K 8/41*** *(2006.01)* ***A61K 8/58*** *(2006.01)*
***A61Q 17/04*** *(2006.01)* ***A61K 8/49*** *(2006.01)*
***A61Q 19/08*** *(2006.01)*

(86) International application number:
**PCT/EP2014/051009**

(87) International publication number:
**WO 2014/111563 (24.07.2014 Gazette 2014/30)**

(54) **COSMETIC OR DERMATOLOGICAL COMPOSITION COMPRISING A MEROCYANINE AND A LIPOPHILIC BENZOTRIAZOLE UV-SCREENING AGENT AND/OR A BIS-RESORCINYL TRIAZINE COMPOUND**

KOSMETISCHE ODER DERMATOLOGISCHE ZUSAMMENSETZUNG MIT EINEM MEROCYANIN UND EINEM LIPOPHILEN BENZOTRIAZOL-UV-ABSCHIRMUNGSMITTEL UND/ODER EINER BISRESORZINYL-TRIAZIN-VERBINDUNG

COMPOSITION COSMÉTIQUE OU DERMATOLOGIQUE COMPRENANT UNE MÉROCYANINE ET UN AGENT ANTI-UV À BASE DE BENZOTRIAZOLE ET/OU UN COMPOSÉ BIS-RÉSORCINYL TRIAZINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:
**21.01.2013 FR 1350487**
**21.01.2013 FR 1350481**
**21.02.2013 US 201361767339 P**
**21.02.2013 US 201361767331 P**

(43) Date of publication of application:
**25.11.2015 Bulletin 2015/48**

(73) Proprietor: **L'Oréal**
**75008 Paris (FR)**

(72) Inventors:
- **ROUDOT, Angelina**
  **F-94270 Le Kremlin Bicêtre (FR)**
- **CANDAU, Didier**
  **F-91570 Bièvres (FR)**

(74) Representative: **Le Blainvaux Bellegarde, Françoise**
**L'Oréal**
**Service DIPI**
**9 Rue Pierre Dreyfus**
**92110 Clichy (FR)**

(56) References cited:
**WO-A1-2011/113718** **WO-A1-2013/010590**
**WO-A2-2008/090066** **WO-A2-2009/027258**
**WO-A2-2013/011094**

- **"Mixtures comprising Cinnamates, Benzotriazoles and Merocyanines", IP.COM JOURNAL, IP.COM INC., WEST HENRIETTA, NY, US, 12 November 2009 (2009-11-12), XP013135311, ISSN: 1533-0001**
- **"Process for producing 3-amino-2-cyclohexan-1-ylidene compounds", IP.COM JOURNAL, IP.COM INC., WEST HENRIETTA, NY, US, 29 April 2009 (2009-04-29), XP013131313, ISSN: 1533-0001**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 945 601 B1

## Description

The present invention relates to a cosmetic or dermatological composition comprising, in a physiologically acceptable support:

a) at least one oily phase and
b) at least one merocyanine compound of formula (I) defined hereinbelow and
c) at least one lipophilic benzotriazole UV-screening agent and/or one bis-resorcinyl triazine compound;

the said composition containing less than 2% of cyclohexasiloxane relative to the total weight of the composition when it contains at least one lipophilic benzotriazole UV-screening agent.

The present invention also relates to a non-therapeutic cosmetic process for caring for and/or making up a keratin material, comprising the application to the surface of the said keratin material of at least one composition according to the invention as defined above.

The invention also relates to a non-therapeutic cosmetic process for limiting the darkening of the skin and/or improving the colour and/or the uniformity of the complexion, comprising the application to the surface of the keratin material of at least one composition as defined previously.

The invention also relates to a non-therapeutic cosmetic process for preventing and/or treating the signs of ageing of a keratin material, comprising the application to the surface of the keratin material of at least one composition as defined previously.

It is known that radiation with wavelengths of between 280 nm and 400 nm permits tanning of the human epidermis and that radiation with wavelengths of between 280 and 320 nm, known as UV-B rays, harms the development of a natural tan. Exposure is also liable to bring about a detrimental change in the biomechanical properties of the epidermis, which is reflected by the appearance of wrinkles, leading to premature ageing of the skin.

It is also known that UV-A rays with wavelengths of between 320 and 400 nm penetrate more deeply into the skin than UV-B rays. UV-A rays cause immediate and persistent browning of the skin. Daily exposure to UV-A rays, even of short duration, under normal conditions can result in damage to the collagen fibres and the elastin, which is reflected by a modification in the microrelief of the skin, the appearance of wrinkles and uneven pigmentation (brown spots, lack of uniformity of the complexion).

Protection against UVA and UVB rays is thus necessary. An efficient photoprotective product should protect against both UVA and UVB radiation.

Many photoprotective compositions have been proposed to date to overcome the effects induced by UVA and/or UVB radiation. They generally contain organic and/or mineral UV-screening agents, which function according to their own chemical nature and according to their own properties by absorption, reflection or scattering of the UV radiation. They generally contain mixtures of liposoluble organic screening agents and/or water-soluble UV-screening agents in combination with metal oxide pigments, such as titanium dioxide or zinc oxide.

Many cosmetic compositions for limiting the darkening of the skin and improving the colour and uniformity of the complexion have been proposed to date. It is well known in the field of antisun products that such compositions may be obtained by using UV-screening agents, and in particular UVB-screening agents. Certain compositions may also contain UVA-screening agents. This screening system should cover UVB protection for the purpose of limiting and controlling the neosynthesis of melanin, which promotes the overall pigmentation, but should also cover UVA protection so as to limit and control the oxidation of the already-existing melanin leading to darkening of the skin colour.

However, it is extremely difficult to find a composition which contains a particular combination of UV-screening agents that would be especially suited to improving the quality of the skin as regards both the colour and its mechanical elasticity properties. This improvement is particularly sought on already-pigmented skin so as not to increase the melanin pigmentary load or the structure of the melanin already present in the skin.

In point of fact, the majority of the organic UV-screening agents consist of aromatic compounds which absorb in the wavelength range between 280 and 370 nm. In addition to their power for screening out sunlight, the desired photoprotective compounds should also have good cosmetic properties, good solubility in the usual solvents and in particular in fatty substances such as oils, and also good chemical stability and good photostability alone or in combination with other UV-screening agents. They should also be colourless or at least have a colour that is cosmetically acceptable to the consumer.

One of the main drawbacks known to date of these antisun compositions is that their systems for screening out UVA and UVB radiation are insufficiently effective against UV rays and in particular against long UVA rays with wavelengths beyond 370 nm, for the purpose of controlling photo-induced pigmentation and its evolution by a UV-screening system on the entire UV spectrum.

In this respect, a particularly advantageous family of UV-A screening agents is currently composed of dibenzoylmethane derivatives and in particular 4-tert-butyl-4'-methoxydibenzoylmethane, this being because they have high

intrinsic absorbing power. These dibenzoylmethane derivatives, which are products that are now well known per se as screening agents that are active in the UV-A range, are described in particular in French patent applications FR-A-2 326 405 and FR-A-2 440 933, and also in European patent application EP-A-0 114 607; 4-tert-butyl-4'-methoxydibenzoylmethane is moreover currently sold under the trade name Parsol 1789® by the company DSM Nutritional Products. However, these compounds are photo-unstable and cannot be combined with UVB-screening agents to afford broad UV protection over the range 280 to 400 nm. In point of fact, these UVA and UVB screening systems thus constituted make it possible to protect from 280 nm to 370 nm, and at best to 380 nm in large amounts.

Benzotriazole screening agents are particularly advantageous since they absorb both UVA and UVB radiation. Mention may be made especially of 2-benzotriazolyl-4-tert-octylphenol or Octrizole (UV absorber-5), 2-(2H-benzotriazol-2-yl)-4-methylphenol or Drometrizole (UV absorber 1), the benzotriazoles sold under the trade names Tinuvin® by the company BASF, especially the lipophilic compound 2-2(H-benzotriazol-2-yl)-p-cresol sold under the trade name Tinuvin P® or the product sold under the name Uvazol P® by the company Enichem Synth.

The following are also known:

- 2-(2'-hydroxy-3'-butyl-5'-methylphenyl)benzotriazole such as the product sold under the name Uvazol 236® by the company Enichem Synth;
- 2-(2'-hydroxy-5'-t-octylphenyl)benzotriazole such as the product sold under the name Uvazol 311® by the company Enichem Synth.

Patent application WO 00/25370 also discloses lipophilic benzotriazole UV-screening agents such as

- benzotriazolyl dodecyl p-cresol sold under the trade name Tinogard TL® by the company BASF,
- 2-t-butyl-6-(5-chloro-2H-benzotriazol-2-yl)-p-cresol (INCI name: Bumetrizole) such as the product sold under the trade name Tinogard AS® by the company BASF.

The ones mentioned in patent US 5 869 030 are especially known. Among these screening agents, mention may be made most particularly of methylenebis(benzotriazolyl)tetramethylbutylphenol sold in solid form under the trade name Mixxim BB/100® by Fairmount Chemical, or in micronized form as an aqueous dispersion under the trade name Tinosorb M® by the company BASF. They have the drawback of being insoluble in oils and in water and give rise to formulation problems in the usual supports for antisun compositions.

Patent application EP 1 977 730 (Shiseido) also discloses a family of benzotriazole of formula

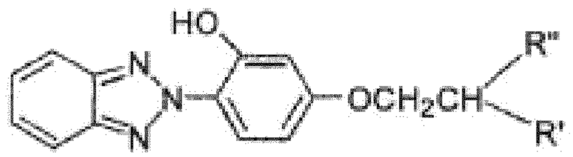

in which R' denotes a linear $C_1$-$C_6$ alkyl and R" denotes a $C_1$-$C_3$ alkyl.

The UVA and UVB screening systems described in the said patent application especially with the UVA-screening agent 4-tert-butyl-4'-methoxydibenzoylmethane containing these lipophilic benzotriazole screening agents do not afford broad UV protection over the range 280 to 400 nm.

Another interesting family of lipophilic benzotriazole screening agents is that of silicones bearing a benzotriazole function as described in patent EP 0 660 701, which also absorb both UVA and UVB radiation. In this same patent, these silicones bearing a benzotriazole function are combined with a dibenzoylmethane UVA-screening agent such as those described previously and the UVA and UVB screening system thus constituted does not afford broad UV protection over the range 280 to 400 nm.

Bis-resorcinyl triazine compounds are particularly advantageous since they absorb both UVA and UVB radiation. These compounds are described, for example, in patent application EP 0 775 698. Mention may be made especially of the derivative 2,4-bis{[4-(2-ethylhexyloxy)-2-hydroxy]phenyl}-6-(4-methoxyphenyl)-1,3,5-triazine, sold under the trade name Tinosorb S by the company BASF.

The UVA and UVB screening systems described in the said patent application especially with the UVA-screening agent 4-tert-butyl-4'-methoxydibenzoylmethane containing these bis-resorcinyl triazine screening agents do not, however, make it possible to afford broad UV protection over the range 280 to 400 nm.

Merocyanine compounds are known in patent US 4 195 999, patent application WO 2004/006 878, patent applications WO2008/090066, WO2011/113718, WO2009/027258, WO2013010590, WO2013/011094, WO20130/11480 and the documents IP COM JOURNAL N°000179675D published on February 23, 2009, IP COM JOURNAL N°000182396D published on April 29, IP COM JOURNAL N° 000189542D published on November 12, 2009,

IP COM Journal N°IPCOM000011179D published on 03/04/2004.

Some of these compounds may show the following drawbacks :

- relatively unsatisfactory solubility in the usual solvents and in particular in fatty substances such as oils which may require a laborious formulation process and/or may result in cosmetic drawbacks such as a greasy effect on application ;
- an unsatisfactory chemical stability and/or unsatisfactory photostability
- produce a color liable to discourage the consumer from using a cosmetic or dermatological composition containing them.

The UVA and UVB screening systems consisting of some of thsed merocyanine screening agents as the compound Octyl-5-N,N-diethylamino-2-phenysulfonyl-2,4-pentadienoate corresponding to the compound MC172 of structure

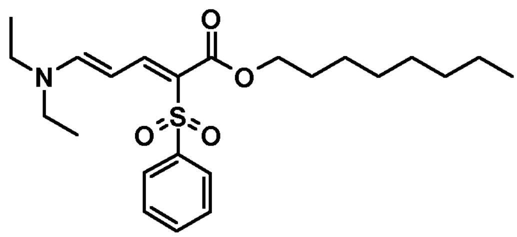

and of lipophilic screening agents such as lipophilic benzotriazole filters and bis-resorcinyl triazine compounds do not always make it possible to afford broad UV protection over the range 280 to 400 nm and especially to obtain an observable absorbance up to a wavelength of 400 nm inclusive.

There thus remains a need to find a novel lipophilic UVA and UVB screening system based on a merocyanine compound and a UV-screening agent of the benzotriazole type and/or a bis-resorcinyl triazine compound which is photostable and which ensures overall protection against UV rays from 280 to 400 nm especially having notable absorbance ranging up to a wavelength of 400 nm inclusive, in a stable manner over time and at high temperatures, without the drawbacks as previously defined.

The Applicant has discovered, surprisingly, that this objective can be achieved by using at least one lipophilic benzotriazole UV-screening agent and/or at least one bis-resorcinyl triazine compound and at least one particular merocyanine of formula (I) which will be defined in greater detail herein below.

Furthermore, the merocyanine compounds of formula (I) herein below, present surprinsingly the advantage to be significantly less colored than the merocyanine compounds as disclosed in the application WO2008/090066 as the compound MC11 also called MC03 in the application WO2009/027258.

Those discoveries form the basis of the present invention.

Thus, in accordance with one of the objects of the present invention, a cosmetic or dermatological composition is now proposed, comprising, in a physiologically acceptable support:

a) at least one oily phase and
b) at least one merocyanine compound of formula (I) defined hereinbelow and

at least one lipophilic benzotriazole UV-screening agent and/or one bis-resorcinyl triazine compound;
the said composition containing less than 2% of cyclohexasiloxane relative to the total weight of the composition when it contains at least one lipophilic benzotriazole UV-screening agent.

Another subject of the present invention consists of a non-therapeutic cosmetic process for caring for and/or making up a keratin material, comprising the application to the surface of the said keratin material of at least one composition according to the invention as defined above.

The invention also relates to a non-therapeutic cosmetic process for limiting the darkening of the skin and/or improving the colour and/or the uniformity of the complexion, comprising the application to the surface of the keratin material of at least one composition as defined previously.

The invention also relates to a non-therapeutic cosmetic process for preventing and/or treating the signs of ageing of a keratin material, comprising the application to the surface of the keratin material of at least one composition as defined previously.

Other characteristics, aspects and advantages of the invention will emerge on reading the detailed description that follows.

The expression "human keratin materials" means the skin (body, face, area around the eyes), hair, eyelashes, eyebrows, body hair, nails, lips or mucous membranes.

The term "physiologically acceptable" means compatible with the skin and/or its integuments, having a pleasant colour, odour and feel and not causing any unacceptable discomfort (stinging, tautness or redness) liable to discourage the consumer from using this composition.

The term "lipophilic screening agent" means any organic or mineral, cosmetic or dermatological compound for screening out UV radiation which can be completely dissolved in molecular form in a liquid aqueous phase or which can be dissolved in colloidal form (for example in micellar form) in a liquid fatty phase.

The term "between X and Y" means the range of values also including the limits X and Y.

According to the invention, the term "preventing" or "prevention" means reducing the risk of occurrence or slowing down the occurrence of a given phenomenon, namely, according to the present invention, the signs of ageing of a keratin material.

## MEROCYANINES

According to the present invention, the merocyanine compounds in accordance with the invention correspond to formula (I) below, and also the E/E- or E/Z-geometrical isomer forms thereof:

(I) 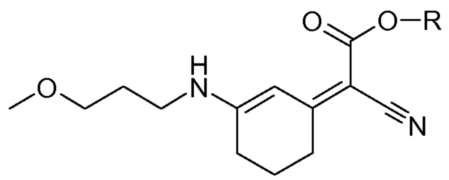

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being substituted with one or more O.

The merocyanine compounds of the invention may be in their E/E- or E/Z-geometrical isomer forms.

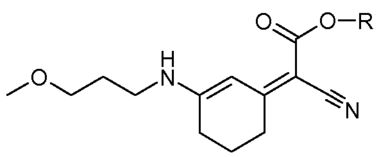 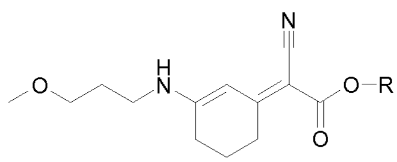

The preferential compounds of formula (I) are those in which:

R is a $C_1$-$C_{22}$ alkyl, which may be substituted with one or more O.

Among the compounds of formula (I), use will be made more particularly of those chosen from the following compounds, and also the E/E- or E/Z- geometrical isomer forms thereof:

| | | | |
|---|---|---|---|
| 1 | 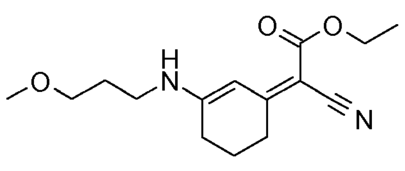<br>ethyl (2Z)-cyano{3-[(3-methoxypropyl)amino] cyclohex-2-en-1-ylidene}ethanoate | 4 | 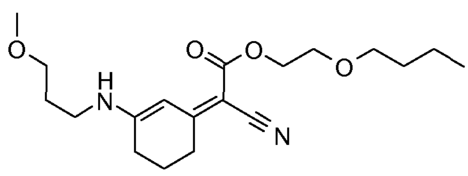<br>2-butoxyethyl (2Z)-cyano{3-[(3-methoxypropyl) amino]cyclohex-2-en-1-ylidene}ethanoate |
| 2 | 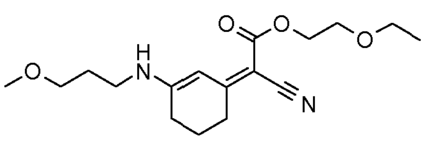<br>2-ethoxyethyl (2Z)-cyano{3-[(3-methoxypropyl) amino]cyclohex-2-en-1-ylidene}ethanoate | 5 | 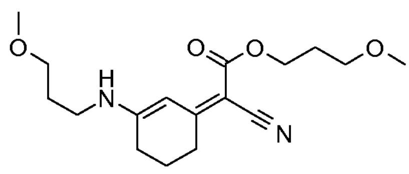<br>3-methoxypropyl (2Z)-cyano{3-[(3-methoxypropyl) amino]cyclohex-2-en-1-ylidene}ethanoate |

(continued)

| | | | |
|---|---|---|---|
| 3 | 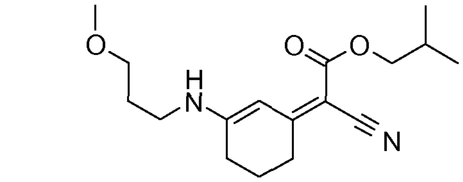<br>2-methylpropyl (2Z)-cyano{3-[(3-methoxypropyl) amino]cyclohex-2-en-1-ylidene}ethanoate | 6 | 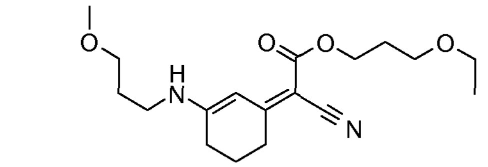<br>3-ethoxypropyl (2Z)-cyano{3-[(3-methoxypropyl) amino]cyclohex-2-en-1-ylidene}ethanoate |

According to a particular mode of the invention, use will be made of those chosen from the following compounds, and also the E/E- or E/Z- geometrical isomer forms thereof:

| | | | |
|---|---|---|---|
| 2 | 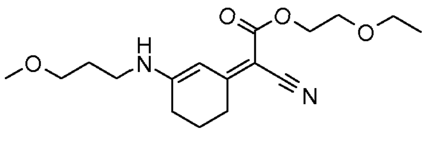<br>2-ethoxyethyl(2Z)-cyano{3-[(3-methoxypropyl) amino]cyclohex-2-en-1-ylidene}ethanoate | 5 | 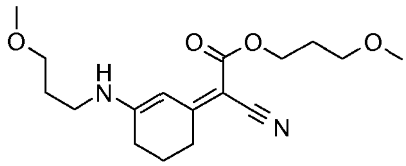<br>3-methoxypropyl(2Z)-cyano{3-[(3-methoxypropyl) amino]cyclohex-2-en-1-ylidene}ethanoate |
| 3 | 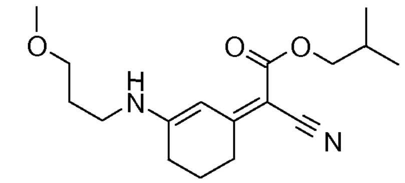<br>2-methylpropyl (2Z)-cyano{3-[(3-methoxypropyl) amino]cyclohex-2-en-1-ylidene}ethanoate | 6 | 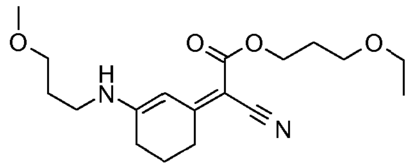<br>3-ethoxypropyl (2Z)-cyano{3-[(3-methoxypropyl) amino]cyclohex-2-en-1-ylidene}ethanoate |
| 4 | 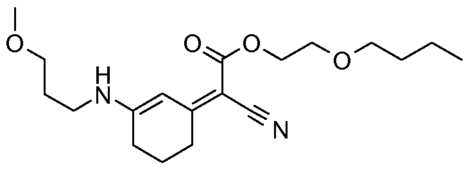<br>2-butoxyethyl (2Z)-cyano{3-[(3-methoxypropyl) amino]cyclohex-2-en-1-ylidene}ethanoate | | |

According to a more particularly preferred mode of the invention, use will be made of the compound 2-ethoxyethyl (2Z)-cyano{3-[(3-methoxypropyl)amino]cyclohex-2-en-1-ylidene}ethanoate (2) in its E/Z geometrical configuration having the following structure:

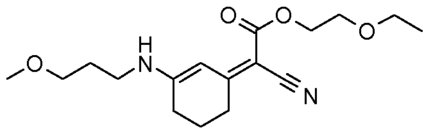

and/or in its E/E geometrical configuration having the following structure:

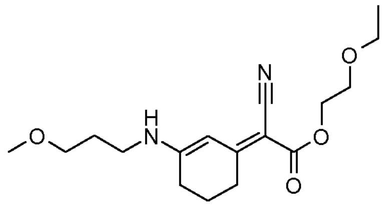

The merocyanines of formula (I) according to the invention are preferably present in the compositions according to the invention in a concentration ranging from 0.1 % to 10% by weight and preferentially from 0.2% to 5% by weight relative to the total weight of the composition.

The compounds of formula (I) may be prepared according to the protocols described in Pat. Appl. WO 2007/071 582, in IP.com Journal (2009), 9(5A), 29-30 IPCOM000182396D under the title "Process for producing 3-amino-2-cyclohexan-1-ylidene compounds" and in US-A-4 749 643 on column 13, line 66 - column 14, line 57 and the references cited in this regard.

## LIPOPHILIC BENZOTRIAZOLES

Among the lipophilic benzotriazole UV-screening agents that may be used according to the invention, mention may be made of

- 2-benzotriazolyl-4-tert-octylphenol or Octrizole (UV absorber-5),
- 2-(2H-benzotriazol-2-yl)-4-methylphenol or Drometrizole (UV absorber 1),
- 2-2(H-benzotriazol-2-yl)-p-cresol sold under the trade name Tinuvin P® by the company BASF or the product sold under the name Uvazol P® by the company Enichem Synth,
- 2-(2'-hydroxy-3'-butyl-5'-methylphenyl)benzotriazole such as the product sold under the trade name Uvazol 236® by the company Enichem Synth,
- 2-(2'-hydroxy-5'-t-octylphenyl)benzotriazole such as the product sold under the trade name Uvazol 311® by the company Enichem Synth,
- benzotriazolyl dodecyl p-cresol such as the product sold under the trade name Tinogard TL® by the company BASF,
- 2-t-butyl-6-(5-chloro-2H-benzotriazol-2-yl)-p-cresol (INCI name: Bumetrizole) such as the product sold under the trade name Tinogard AS® by the company BASF,
- benzotriazole compounds such as those described in patent application EP 1 977 730 (Shiseido), a family of benzotriazole of formula

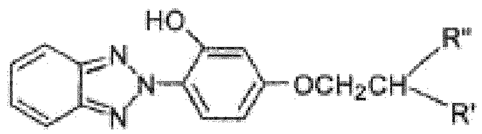

in which R' denotes a linear $C_1$-$C_6$ alkyl and R" denotes a $C_1$-$C_3$ alkyl, in particular 2-[4-(2-ethylhexyloxy)-2-hydroxyphenyl]-2H-benzotriazole and 2-(2-hydroxy-4-isobutoxyphenyl)-2H-benzotriazole, and mixtures thereof.

A particularly preferred family of lipophilic benzotriazoles comprises silanes or siloxanes bearing a benzotriazole function comprising at least one unit of formula (1) below:

$$O_{(3-a)/2}Si(R_7)_a\text{-}G \qquad (1)$$

in which:

- $R_7$ represents an optionally halogenated $C_1$-$C_{10}$ alkyl radical or a phenyl radical or a trimethylsiloxy radical,
- a is an integer chosen between 0 and 3 inclusive,
- and the symbol G denotes a monovalent radical directly bonded to a silicon atom, and which corresponds to formula (2) below:

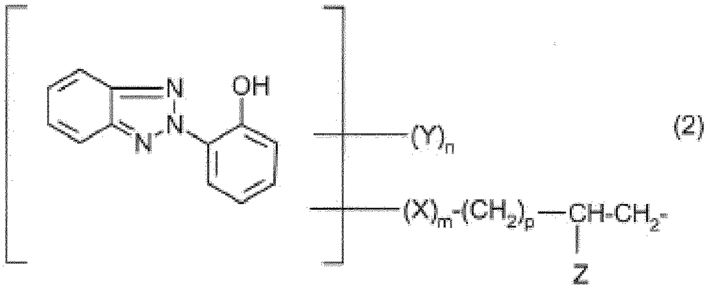

(2)

in which:

- Y, which may be identical or different, are chosen from $C_1$-$C_8$ alkyl radicals, halogens and $C_1$-$C_4$ alkoxy radicals, it being understood that, in the latter case, two adjacent Y of the same aromatic nucleus may together form an alkylidenedioxy group in which the alkylidene group contains from 1 to 2 carbon atoms,
- X represents O or NH,
- Z represents hydrogen or a $C_1$-$C_4$ alkyl radical,
- n is an integer between 0 and 3 inclusive,
- m is 0 or 1,
- p represents an integer between 1 and 10 inclusive.

These compounds are especially described in patent applications EP-A-0 392 883; EP-A-0 660 701; EP-A-0 708 108; EP-A-0 711 778; EP-A-711 779.

Preferably, the silicon derivatives used in the context of the present invention belong to the general family of benzotriazole silicones that is described especially in EP-A-0 660 701.

A family of benzotriazole silicones that is particularly suitable for performing the present invention is that combining the compounds corresponding to formula (5) or (6) below:

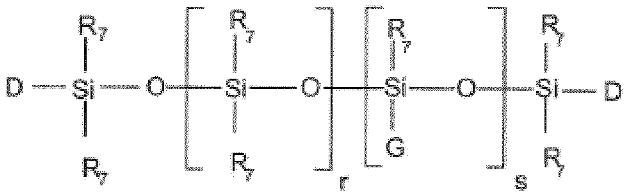

(5)

or

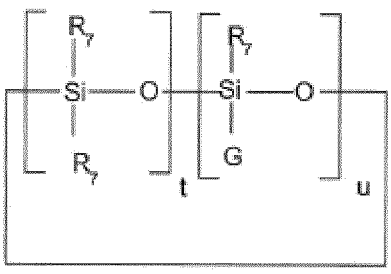

(6)

in which:

- $R_7$, which may be identical or different, are chosen from $C_1$-$C_{10}$ alkyl, phenyl, 3,3,3-trifluoropropyl and trimethylsiloxy radicals, at least 80% by number of the radicals $R_7$ being methyl,
- D, which may be identical or different, are chosen from the radicals $R_7$ and the radical G,
- r is an integer between 0 and 50 inclusive, and s is an integer between 0 and 20 inclusive, and if s = 0, at least one of the two symbols D denotes G,
- u is an integer between 1 and 6 inclusive, and t is an integer between 0 and 10 inclusive, it being understood that t + u is equal to or greater than 3,

- and the symbol G corresponds to formula (2) above.

As emerges from formula (2) given above, the attachment of the chain unit $-(X)_m-(CH_2)_p-CH(Z)-CH_2-$ to the benzotriazole unit, which thus provides the bonding of the said benzotriazole unit to the silicon atom of the silicone chain, may, according to the present invention, take place in any of the available positions offered by the two aromatic nuclei of the benzotriazole:

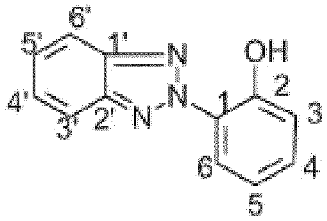

Preferably, this attachment takes place in position 3, 4, 5 (aromatic nucleus bearing the hydroxyl function) or 4' (benzene nucleus adjacent to the triazole ring), and even more preferentially in position 3, 4 or 5. In a preferred embodiment of the invention, the attachment takes place in position 3.

Similarly, the attachment of the substituent unit(s) Y may take place in any of the other available positions on the benzotriazole. However, preferably, this attachment takes place in position 3, 4, 4', 5 and/or 6. In a preferred embodiment of the invention, the attachment of the unit Y takes place in position 5.

In formulae (5) and (6) above, the alkyl radicals may be linear or branched and chosen especially from methyl, ethyl, n-propyl, isopropyl, n-butyl, isobutyl, tert-butyl, n-amyl, isoamyl, neopentyl, n-hexyl, n-heptyl, n-octyl, 2-ethylhexyl and tert-octyl radicals. The alkyl radicals $R_7$ that are preferred according to the invention are the methyl, ethyl, propyl, n-butyl, n-octyl and 2-ethylhexyl radicals. Even more preferentially, the radicals $R_7$ are all methyl radicals.

Among the compounds of formula (5) or (6) above, it is preferred to use those corresponding to formula (5), i.e. diorganosiloxanes bearing a short linear chain.

Among the compounds of formula (5) above, it is preferred to use those for which the radicals D are both radicals $R_7$.

Among the linear diorganosiloxanes of formula (5) included in the context of the present invention, the ones that are more particularly preferred are the random derivatives or the derivatives in well-defined blocks having at least one and even more preferentially all of the following characteristics:

- D is a radical $R_7$,
- $R_7$ is alkyl and even more preferentially is methyl,
- r is between 0 and 15 inclusive; s is between 1 and 10 inclusive,
- n is non-zero and preferably equal to 1, and Y is then chosen from methyl, tert-butyl and $C_1$-$C_4$ alkoxy,
- Z is hydrogen or methyl,
- m = 0, or [m = 1 and X = O],
- p is equal to 1.

A family of benzotriazole silicones that is particularly suitable for use in the invention is that defined by the general formula (7) below:

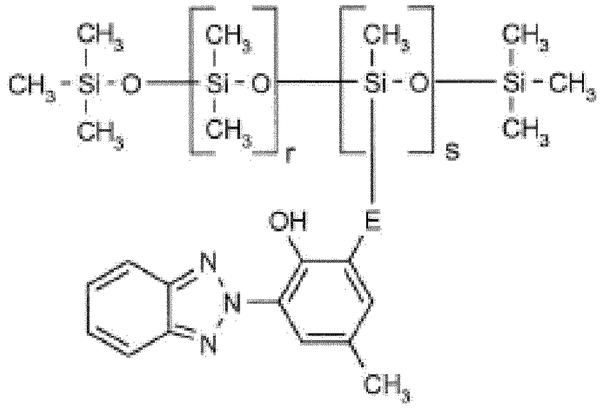

(7)

with $0 \leq r \leq 10$,
$1 \leq s \leq 10$,
and in which E represents the divalent radical:

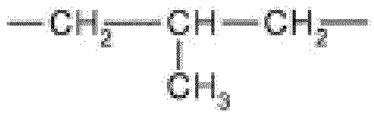

In a particularly preferred embodiment of the invention, the benzotriazole silicone is the compound (referred to as compound (a) in the rest of the text) corresponding to the following formula:

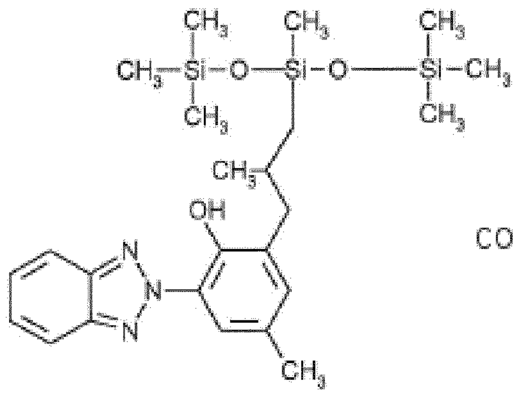

compound (a)

Processes that are suitable for preparing the products of formulae (1), (5), (6) and (7) above are especially described in the American patents US 3 220 972, US 3 697 473, US 4 340 709, US 4 316 033, US 4 328 346 and in patent applications EP-A-0 392 883 and EP-A-0 742 003.

The lipophilic benzotriazole screening agent(s) are preferably present in the compositions according to the invention in contents ranging from 0.1% to 20% by weight and better still ranging from 0.2% to 15% by weight, always relative to the total weight of the composition.

## BIS-RESORCINYL TRIAZINE COMPOUNDS

The bis-resorcinyl triazine compounds in accordance with the present invention correspond to formula (II) below:

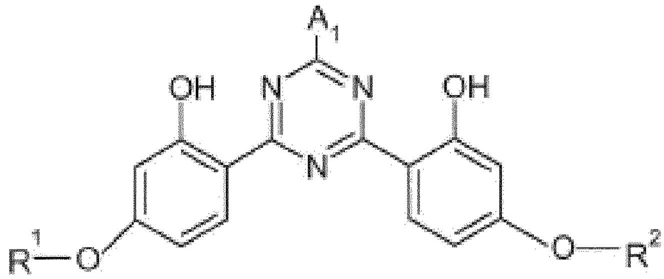

(II)

in which:

(i) the radicals $R^1$ and $R^2$, which may be identical or different, denote a $C_3$-$C_{18}$ alkyl radical; a $C_2$-$C_{18}$ alkenyl radical or a residue of formula $-CH_2-CH(OH)-CH_2-OT_1$ in which $T_1$ is a hydrogen atom or a $C_1$-$C_8$ alkyl radical; or
(ii) the radicals $R^1$ and $R^2$, which may be identical or different, denote a residue of formula (III) below:

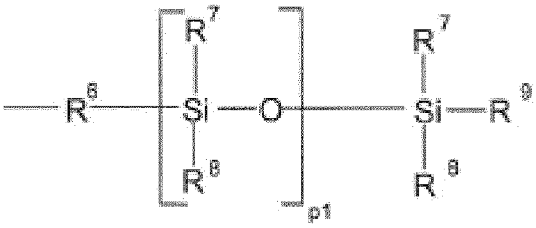

(III)

in which:

- $R^6$ denotes a covalent bond; a linear or branched $C_1$-$C_4$ alkylene radical or a residue of formula $-C_{m1}H_{2m1}-$ or $-C_{m1}H_{2m1}-O-$ in which m1 is a number from 1 to 4;
- p1 is a number from 0 to 5;
- the radicals $R^7$, $R^8$ and $R^9$, which may be identical or different, denote a $C_1$-$C_{18}$ alkyl radical; a $C_1$-$C_{18}$ alkoxy radical or a residue of formula:

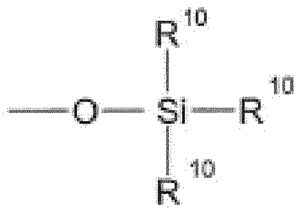

(IV)

in which $R^{10}$ is a $C_1$-$C_5$ alkyl radical;

- $A_1$ denotes a residue corresponding to one of the following formulae:

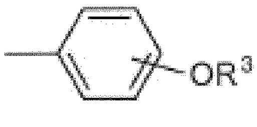

(V)

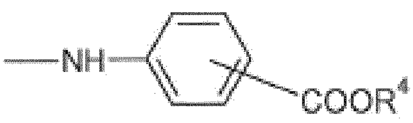

(VI)

10

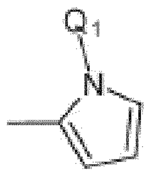

(VII)

in which:

- $R^3$ denotes a hydrogen atom, a $C_1$-$C_{10}$ alkyl radical or a radical of formula: $-(CH_2CHR^5-O)_{n1}R^4$ in which n1 is a number from 1 to 16,
- $R^4$ denotes hydrogen, a metal cation M, a $C_1$-$C_5$ alkyl radical or a residue of formula $-(CH_2)_{m2}-OT_1$ in which m2 is a number from 1 to 4 and $T_1$ has the same meaning indicated in paragraph (i),
- $R^5$ is hydrogen or methyl, or a residue of structure $-CH_2-CH-(OH)-CH_2OT_1$ with $T_1$ having the same meaning indicated in paragraph (i),
- $Q_1$ is a $C_1$-$C_{18}$ alkyl radical.

In formulae (II) and (III) to (VI) described above:

- the alkyl radicals are linear or branched and may be chosen, for example, from methyl, ethyl, n-propyl, isopropyl, n-butyl, sec-butyl, tert-butyl, amyl, isoamyl, tert-amyl, heptyl, octyl, isooctyl, nonyl, decyl, undecyl, dodecyl, tetradecyl, pentadecyl, hexadecyl, heptadecyl and octadecyl;
- the alkenyl radicals may be chosen, for example, from allyl, methallyl, isopropenyl, 2-butenyl, 3-butenyl, isobutenyl, n-penta-2,4-dienyl, 3-methyl-2-butenyl, n-oct-2-enyl, n-dodec-2-enyl, isododecenyl and n-octadec-4-enyl;
- the alkoxy radicals are linear or branched and may be chosen, for example, from methoxy, ethoxy, n-propoxy, isopropoxy, n-butoxy, sec-butoxy, tert-butoxy, amyloxy, isoamyloxy and tert-amyloxy;
- the $C_1$-$C_5$ monoalkylamino or dialkylamino radicals may be chosen, for example, from methylamino, ethylamino, propylamino, n-butylamino, sec-butylamino, tert-butylamino, pentylamino, dimethylamino, diethylamino, dibutylamino and methylethylamino,
- the metal cations are alkali metal, alkaline-earth metal or metal cations chosen, for example, from lithium, potassium, sodium, calcium, magnesium, copper and zinc.

The bis-resorcinyl triazine derivatives of formula (II) of the invention are screening agents that are already known per se. They are described and prepared according to the syntheses indicated in patent application EP-A-0 775 698.

As examples of compounds of formula (II) that may be used, mention may be made of:

- 2,4-bis{[4-(2-ethylhexyloxy)-2-hydroxy]phenyl}-6-(4-methoxyphenyl)-1,3,5-triazine;
- 2,4-bis{[4-(3-(2-propyloxy)-2-hydroxypropyloxy)-2-hydroxy]phenyl}-6-(4-methoxyphenyl)-1,3,5-triazine;
- 2,4-bis{[4-(2-ethylhexyloxy)-2-hydroxy]phenyl}-6-[4-(2-methoxyethyl-carboxyl)phenylamino]-1,3,5-triazine;
- 2,4-bis{[4-tris(trimethylsiloxysilylpropyloxy)-2-hydroxy]phenyl}-6-(4-methoxyphenyl)-1,3,5-triazine;
- 2,4-bis{[4-(2''-methylpropenyloxy)-2-hydroxy]phenyl}-6-(4-methoxyphenyl)-1,3,5-triazine;
- 2,4-bis{[4-(1',1',1',3',5',5',5'-heptamethyltrisiloxy-2''-methylpropyloxy)-2-hydroxy]phenyl}-6-(4-methoxyphenyl)-1,3,5-triazine;
- 2,4-bis{[4-(3-(2-propyloxy)-2-hydroxypropyloxy]-2-hydroxy]phenyl}-6-[(4-ethylcarboxy)phenylamino]-1,3,5-triazine;
- 2,4-bis{[4-(2-ethylhexyloxy)-2-hydroxy]phenyl}-6-(1-methyl-2-pyrrolyl)-1,3,5-triazine, and mixtures thereof.

The bis-resorcinyl triazine derivative compounds that are more particularly preferred according to the invention are chosen from the group consisting of:

- 2,4-bis{[4-(2-ethylhexyloxy)-2-hydroxy]phenyl}-6-(4-methoxyphenyl)-1,3,5-triazine;
- 2,4-bis{[4-tris(trimethylsiloxysilylpropyloxy)-2-hydroxy]phenyl}-6-(4-methoxyphenyl)-1,3,5-triazine;
- 2,4-bis{[4-(1',1',1',3',5',5',5'-heptamethyltrisiloxy-2''-methylpropyloxy)-2-hydroxy]phenyl}-6-(4-methoxyphenyl)-1,3,5-triazine.

The bis-resorcinyl triazine compound that is more particularly preferred according to the invention will be the compound 2,4-bis{[4-(2-ethylhexyloxy)-2-hydroxy]phenyl}-6-(4-methoxyphenyl)-1,3,5-triazine (INCI name: Bis-ethylhexyloxyphenol methoxyphenyl triazine) sold under the trade name Tinosorb S by BASF.

The bis-resorcinyl triazine derivative compounds are preferably present in the compositions in accordance with the invention in contents ranging from 0.01% to 20% by weight, more preferentially from 0.1% to 10% by weight and even more preferentially from 0.1% to 6% by weight relative to the total weight of the composition.

## OILY PHASE

The compositions in accordance with the invention comprise at least one oily phase.

The compositions of the invention containing at least one lipophilic benzotriazole UV-screening agent contain less than 2% of cyclohexasiloxane relative to the total weight of the composition, since it may pose incompatibility problems with certain oils usually used in antisun formulations.

For the purposes of the invention, the term "oily phase" is understood to mean a phase comprising at least one oil and all of the liposoluble and lipophilic ingredients and the fatty substances used for the formulation of the compositions of the invention.

The term "oil" is understood to mean any fatty substance in the liquid form at room temperature (20 - 25°C) and at atmospheric pressure (760 mmHg).

An oil suitable for the invention can be volatile or non-volatile.

An oil suitable for the invention can be chosen from hydrocarbon-based oils, silicone oils, fluorinated oils and mixtures thereof.

A hydrocarbon-based oil suitable for the invention can be an animal hydrocarbon-based oil, a vegetable hydrocarbon-based oil, a mineral hydrocarbon-based oil or a synthetic hydrocarbon-based oil.

An oil suitable for the invention can advantageously be chosen from mineral hydrocarbon-based oils, vegetable hydrocarbon-based oils, synthetic hydrocarbon-based oils, silicone oils and mixtures thereof.

For the purposes of the present invention, the term "silicone oil" means an oil comprising at least one silicon atom, and especially at least one Si-O group.

The term "hydrocarbon-based oil" is understood to mean an oil comprising mainly hydrogen and carbon atoms.

The term "fluorinated oil" is understood to mean an oil comprising at least one fluorine atom.

A hydrocarbon-based oil suitable for the invention can in addition optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent or agents, at least one volatile or non-volatile hydrocarbon-based oil and/or one volatile and/or non-volatile silicone oil.

For the purposes of the invention, the term "volatile oil" means an oil that is capable of evaporating on contact with the skin or the keratin fibre in less than one hour, at room temperature and atmospheric pressure. The volatile oil(s) of the invention are volatile cosmetic oils which are liquid at room temperature and which have a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is understood to mean an oil which remains on the skin or the keratinous fibre, at room temperature and atmospheric pressure, for at least several hours and which has in particular a vapour pressure of less than $10^{-3}$ mmHg (0.13 Pa).

### Hydrocarbon-based oils

Mention may in particular be made, as non-volatile hydrocarbon-based oils which can be used according to the invention, of:

(i) hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheatgerm oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin seed oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stéarineries Dubois or those sold under the names Miglyol 810®, 812® and 818® by Dynamit Nobel,

(ii) synthetic ethers having from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, for instance oils of formula RCOOR' in which R represents a linear or branched fatty acid residue containing from 1 to 40 carbon atoms and R' represents a hydrocarbon-based chain that is especially branched, containing from 1 to 40 carbon atoms on condition that
R + R' is ≥10, for instance purcellin oil (cetearyl octanoate), isopropyl myristate, isopropyl palmitate, $C_{12}$-$C_{15}$ alkyl benzoate, such as the product sold under the trade name Finsolv TN® or Witconol TN® by Witco or Tegosoft TN® by Evonik Goldschmidt, 2-ethylphenyl benzoate, such as the commercial product sold under the name X-Tend 226® by ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI® by Enichem Augusta Industriale, and also di(linear $C_{14}$-$C_{15}$ alkyl) tartrates, such as those sold under the name Cosmacol ETL® by the same company; or acetates;

(v) fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates, such as dicaprylyl carbonate, such as the product sold under the name Cetiol CC® by Cognis;

(viii) fatty amides, such as isopropyl N-lauroyl sarcosinate, such as the product sold under the trade name Eldew SL205® from Ajinomoto;

and mixtures thereof.

Preference will more particularly be given, among the non-volatile hydrocarbon oils which can be used according to the invention, to glyceride triesters and in particular to caprylic/capric acid triglycerides, synthetic esters and in particular isononyl isononanoate, oleyl erucate, $C_{12}$-$C_{15}$ alkyl benzoate, 2-ethylphenyl benzoate and fatty alcohols, in particular octyldodecanol.

Mention may in particular be made, as volatile hydrocarbon oils which can be used according to the invention, of hydrocarbon oils having from 8 to 16 carbon atoms and in particular of branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched $C_8$-$C_{16}$ esters, isohexyl neopentanoate, and mixtures thereof.

Mention may also be made of the alkanes described in the patent applications of Cognis WO 2007/068371 or WO 2008/155059 (mixtures of distinct alkanes differing by at least one carbon). These alkanes are obtained from fatty alcohols, themselves obtained from coconut or palm oil. Mention may be made of the mixtures of n-undecane ($C_{11}$) and n-tridecane ($C_{13}$) obtained in Examples 1 and 2 of patent application WO2008/155059 from Cognis. Mention may also be made of n-dodecane ($C_{12}$) and n-tetradecane ($C_{14}$), sold by Sasol respectively under the references Parafol 12-97® and Parafol 14-97®, and also their mixtures.

Use may also be made of other volatile hydrocarbon oils, such as petroleum distillates, in particular those sold under the name Shell Solt® by Shell. According to one embodiment, the volatile solvent is chosen from volatile hydrocarbon-based oils having from 8 to 16 carbon atoms, and mixtures thereof.

Silicone oils

The non-volatile silicone oils can be chosen in particular from non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicones, especially those with a
viscosity ≤ 8 centistokes ($8 \times 10^{-6}$ $m^2/s$) and especially containing from 2 to 7 silicon atoms, these silicones optionally comprising alkyl or alkoxy groups containing from 1 to 10 carbon atoms. As volatile silicone oils that may be used in the

invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I):

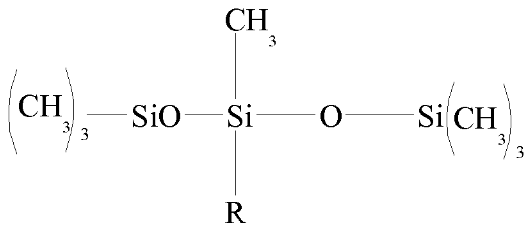

where R represents an alkyl group comprising from 2 to 4 carbon atoms, one or more hydrogen atoms of which can be replaced by a fluorine or chlorine atom.

Mention may be made, among the oils of general formula (I), of:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

Fluorinated oils

Use may also be made of volatile fluorinated oils, such as nonafluoromethoxybutane, decafluoropentane, tetradecafluorohexane, dodecafluoropentane, and mixtures thereof.

An oily phase according to the invention can additionally comprise other fatty substances, mixed with or dissolved in the oil.

Another fatty substance which can be present in the oily phase can be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

According to a specific form of the invention, the overall oily phase, including all the lipophilic substances of the composition capable of being dissolved in this same phase, represents from 5% to 95% by weight and preferably from 10% to 80% by weight, relative to the total weight of the composition.

## AQUEOUS PHASE

The compositions according to the invention can in addition comprise at least one aqueous phase.

The aqueous phase comprises water and optionally other water-soluble or water-miscible organic solvents.

An aqueous phase suitable for the invention can comprise, for example, a water chosen from a natural spring water, such as water from La Roche-Posay, water from Vittel or waters from Vichy, or a floral water.

The water-soluble or water-miscible solvents that are suitable for use in the invention comprise short-chain monoalcohols, for example $C_1$-$C_4$ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.

According to a preferred embodiment, use may more particularly be made of ethanol, propylene glycol, glycerol, and mixtures thereof.

According to a specific form of the invention, the overall aqueous phase, including all the hydrophilic substances

of the composition capable of being dissolved in this same phase, represents from 5% to 95% by weight and preferably from 10% to 80% by weight, relative to the total weight of the composition.

## ADDITIVES

### a) Additional UV-screening agents

The compositions according to the invention may also contain one or more additional UV-screening agents chosen from hydrophilic, lipophilic or insoluble organic UV-screening agents and/or one or more mineral pigments. It will preferentially consist of at least one hydrophilic, lipophilic or insoluble organic UV-screening agent.

The term "hydrophilic UV-screening agent" means any cosmetic or dermatological organic or mineral compound for screening out UV radiation, which can be fully dissolved in molecular state in a liquid aqueous phase or which can be dissolved in colloidal form (for example in micellar form) in a liquid aqueous phase.

The term "lipophilic screening agent" means any cosmetic or dermatological organic or mineral compound for screening out UV radiation, which can be fully dissolved in molecular state in a liquid fatty phase or which can be dissolved in colloidal form (for example in micellar form) in a liquid fatty phase.

The term "insoluble UV-screening agent" means any cosmetic or dermatological organic or mineral compound for screening out UV radiation which has a solubility in water of less than 0.5% by weight and a solubility of less than 0.5% by weight in the majority of organic solvents such as liquid paraffin, fatty alkyl benzoates and fatty acid triglycerides, for example Miglyol 812® sold by the company Dynamit Nobel. This solubility, determined at 70°C, is defined as the amount of product in solution in the solvent at equilibrium with an excess of solid in suspension after returning to room temperature. It may be readily evaluated in the laboratory.

The additional organic UV-screening agents are chosen in particular from cinnamic compounds; anthranilate compounds; salicylic compounds; benzylidenecamphor compounds; benzophenone compounds; β,β-diphenylacrylate compounds; triazine compounds other than bis-resorcinyl triazine compounds; benzotriazole compounds; benzalmalonate compounds, in particular those cited in patent US 5 624 663; benzimidazole derivatives; imidazoline compounds; bis-benzazolyl compounds, such as described in patents EP 669 323 and US 2 463 264; p-aminobenzoic acid (PABA) compounds; methylenebishydroxy(phenyl benzotriazole) compounds as described in applications US 5 237 071, US 5 166 355, GB 2 303 549, DE 197 26 184 and EP 893 119; benzoxazole compounds, such as described in patent applications EP 0 832 642, EP 1 027 883, EP 1 300 137 and DE 101 62 844; screening polymers and screening silicones, such as those described in particular in patent application WO 93/04665; dimers derived from α-alkylstyrene, such as those described in patent application DE 198 55 649; 4,4-diarylbutadiene compounds, such as described in patent applications EP 0 967 200, DE 197 46 654, DE 197 55 649, EP-A-1 008 586, EP 1 133 980 and EP 133 981, and mixtures thereof.

Mention may be made, as examples of organic photoprotective agents, of those denoted hereinbelow under their INCI names:

Cinnamic compounds:

Ethylhexyl Methoxycinnamate, sold in particular under the trade name Parsol MCX® by DSM Nutritional Products,
Isopropyl Methoxycinnamate,
Isoamyl p-methoxycinnamate sold under the trade name Neo Heliopan E 1000® by Symrise,
DEA Methoxycinnamate,
Diisopropyl Methyl Cinnamate,
Glyceryl Ethylhexanoate Dimethoxycinnamate.

para-Aminobenzoic compounds:

PABA,
Ethyl PABA,
Ethyl Dihydroxypropyl PABA,
Ethylhexyl Dimethyl PABA, sold in particular under the name Escalol 507® by ISP, Glyceryl PABA,
PEG-25 PABA, sold under the name Uvinul P 25® by BASF.

Salicylic compounds:

Homosalate, sold under the name Eusolex HMS® by Rona/EM Industries, Ethylhexyl Salicylate, sold under the name Neo Heliopan OS® by Symrise, Dipropylene Glycol Salicylate, sold under the name Dipsal® by Scher, TEA Salicylate, sold under the name Neo Heliopan TS® by Symrise.

β,β-Diphenylacrylate compounds:

Octocrylene, sold in particular under the trade name Uvinul N 539® by BASF, Etocrylene, sold in particular under the trade name Uvinul N 35® by BASF.

Benzophenone compounds:

Benzophenone-1, sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF,
Benzophenone-5,
Benzophenone-6, sold under the trade name Helisorb 11® by Norquay,
Benzophenone-8, sold under the trade name Spectra-Sorb UV-24® by American Cyanamid,
Benzophenone-9, sold under the trade name Uvinul DS 49® by BASF, Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by BASF,
1,1'-(1,4-piperazinediyl)bis[1-[2-[4-(diethylamino)-2-hydroxybenzoyl]phenyl]methanone (CAS 919803-06-8) as described in patent application WO 2007/071 584; this compound being advantageously used in micronized form (mean size from 0.02 to 2 μm) which may be obtained, for example, according to the micronization process described in patent applications GB-A-2 303 549 and EP-A-893 119 and especially in the form of an aqueous dispersion.

Benzylidenecamphor compounds:

3-Benzylidene Camphor, manufactured under the name Mexoryl SD® by Chimex, 4-Methylbenzylidene Camphor, sold under the name Eusolex 6300® by Merck, Benzylidene Camphor Sulfonic Acid, manufactured under the name Mexoryl SL® by Chimex,
Camphor Benzalkonium Methosulfate, manufactured under the name Mexoryl SO® by Chimex,
Terephthalylidene Dicamphor Sulfonic Acid, manufactured under the name Mexoryl SX® by Chimex,
Polyacrylamidomethyl Benzylidene Camphor, manufactured under the name Mexoryl SW® by Chimex.

Phenylbenzimidazole compounds:

Phenylbenzimidazole Sulfonic Acid, sold in particular under the trade name Eusolex 232® by Merck.

Bis-benzazolyl compounds:

Disodium Phenyl Dibenzimidazole Tetrasulfonate, sold under the trade name Neo Heliopan AP® by Haarmann and Reimer.

Phenylbenzotriazole compounds:

Drometrizole Trisiloxane, sold under the name Silatrizole® by Rhodia Chimie.

Methynebis(hyroxyhenybenzotriazole) compounds

Methylenebis(benzotriazolyl)tetramethylbutylphenol especially in solid form, for instance the product sold under the trade name Mixxim BB/100® by the company Fairmount Chemical or in the form of an aqueous dispersion of

micronized particles with a mean particle size ranging from 0.01 to 5 μm, more preferentially from 0.01 to 2 μm and more particularly from 0.020 to 2 μm with at least one alkylpolyglycoside surfactant of structure $C_nH_{2n+1}OC_6H_{10}O_5)_xH$ in which n is an integer from 8 to 16 and x is the mean degree of polymerization of the unit ($C_6H_{10}O_5$) and ranges from 1.4 to 1.6 as described in patent GB-A-2 303 549, sold especially under the trade name Tinosorb M® by the company BASF or in the form of an aqueous dispersion of micronized particles with a mean particle size ranging from 0.02 to 2 μm, more preferentially from 0.01 to 1.5 μm and more particularly from 0.02 to 1 μm in the presence of at least one mono($C_8$-$C_{20}$)alkyl ester of polyglycerol having a degree of glycerol polymerization of at least 5, such as the aqueous dispersions described in patent application WO 2009/063 392.

Triazine compounds:

Ethylhexyl Triazone, sold in particular under the trade name Uvinul T150® by BASF,
Diethylhexyl Butamido Triazone, sold under the trade name Uvasorb HEB® by Sigma 3V,
2,4,6-tris(dineopentyl 4'-aminobenzalmalonate)-s-triazine,
2,4,6-tris(diisobutyl 4'-aminobenzalmalonate)-s-triazine,
2,4-bis(n-butyl 4'-aminobenzoate)-6-(aminopropyltrisiloxane)-s-triazine,
2,4-bis(dineopentyl 4'-aminobenzalmalonate)-6-(n-butyl 4'-aminobenzoate)-s-triazine,
symmetrical triazine screening agents substituted with naphthalenyl groups or polyphenyl groups described in patent US 6 225 467, patent application WO 2004/085 412 (see compounds 6 and 9) or the document Symmetrical Triazine Derivatives IP.COM IPCOM000031257 Journal, INC West Henrietta, NY, US (20 September 2004), especially 2,4,6-tris(diphenyl)triazine and 2,4,6-tris(terphenyl)triazine, which is reviewed in patent applications WO 06/035 000, WO 06/034 982, WO 06/034 991, WO 06/035 007, WO 2006/034 992 and WO 2006/034 985, these compounds being advantageously used in micronized form (mean particle size from 0.02 to 3 μm) which may be obtained, for example, according to the micronization process described in patent applications GB-A-2 303 549 and EP-A-893 119 and especially in aqueous dispersion form,
silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl Anthranilate, sold under the trade name Neo Heliopan MA® by Symrise.

Imidazoline compounds:

Ethylhexyl dimethoxybenzylidene dioxoimidazoline propionate.

Benzalmalonate compounds:

Polyorganosiloxane comprising benzalmalonate functional groups, such as Polysilicone-15, sold under the trade name Parsol SLX® by Hoffmann-LaRoche.

4,4-Diarylbutadiene compounds:

1,1-Dicarboxy(2,2'-dimethylpropyl)-4,4-diphenylbutadiene.

Benzoxazole compounds:

2,4-Bis[4-[5-(1,1-dimethylpropyl)benzoxazol-2-yl]phenylimino]-6-[(2-ethylhexyl)imino]-1,3,5-triazine, sold under the name of Uvasorb K2A® by Sigma 3V.
The preferred organic screening agents are chosen from:

Ethylhexyl methoxycinnamate,
Ethylhexyl salicylate,
Homosalate,
Octocrylene,

Phenylbenzimidazole Sulfonic Acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
Methylenebis(benzotriazolyl)tetramethylbutylphenol,
Ethylhexyl Triazone,
Diethylhexyl Butamido Triazone,
2,4,6-tris(Dineopentyl 4'-aminobenzalmalonate)-s-triazine,
2,4,6-Tris(diisobutyl4'-aminobenzalmalonate)-s-triazine,
2,4-Bis(n-butyl4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
2,4,6-tris(diphenyl)triazine,
2,4,6-tris(terphenyl)triazine,
Polysilicone-15,
1,1-Dicarboxy(2,2'-dimethylpropyl)-4,4-diphenylbutadiene,
2,4-Bis[4-[5-(1,1-dimethylpropyl)benzoxazol-2-yl]phenylimino]-6-[(2-ethylhexyl)imino]-1,3,5-triazine,

and mixtures thereof.

The particularly preferred organic screening agents are chosen from:

Ethylhexyl salicylate,
Homosalate,
Octocrylene,
n-Hexyl 2-(4-diethylamino-2-hydroxybenzoyl)benzoate,
Terephthalylidenedicamphorsulfonic acid,
Ethylhexyl Triazone,
Diethylhexyl Butamido Triazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,

and mixtures thereof.

The mineral UV-screening agents used in accordance with the present invention are metal oxide pigments. More preferentially, the mineral UV-screening agents of the invention are metal oxide particles with a mean elementary particle size of less than or equal to 0.5 μm, more preferentially between 0.005 and 0.5 μm, even more preferentially between 0.01 and 0.2 μm, better still between 0.01 and 0.1 μm and more particularly preferentially between 0.015 and 0.05 μm.

They may be selected in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100

SA® from the company Tayca and Tioveil from the company Tioxide,
- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
- with silica, alumina and alginic acid, such as the product MT-100 AQ® from the company Tayca,
- with alumina and aluminium laurate, such as the product Microtitanium Dioxide MT 100 S® from the company Tayca,
- with iron oxide and iron stearate, such as the product Microtitanium Dioxide MT 100 F® from the company Tayca,
- with zinc oxide and zinc stearate, such as the product BR 351® from the company Tayca,
- with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
- with silica, alumina and aluminium stearate and treated with a silicone, such as the product STT-30-DS® from the company Titan Kogyo,
- with silica and treated with a silicone, such as the product UV-Titan X 195® from the company Sachtleben Pigments,
- with alumina and treated with a silicone, such as the products Tipaque TTO-55 (S)® from the company Ishihara or UV Titan M 262® from the company Sachtleben Pigments,
- with triethanolamine, such as the product STT-65-S from the company Titan Kogyo,
- with stearic acid, such as the product Tipaque TTO-55 (C)® from the company Ishihara,
- with sodium hexametaphosphate, such as the product Microtitanium Dioxide MT 150 W® from the company Tayca.
- $TiO_2$ treated with octyltrimethylsilane, sold under the trade name T 805® by the company Degussa Silices,
- $TiO_2$ treated with a polydimethylsiloxane, sold under the trade name 70250 Cardre UF Ti02SI3® by the company Cardre,
- anatase/rutile $TiO_2$ treated with a polydimethylhydrosiloxane, sold under the trade name Microtitanium Dioxide USP Grade Hydrophobic® by the company Color Techniques.

Mention may also be made of $TiO_2$ pigments doped with at least one transition metal such as iron, zinc or manganese and more particularly manganese. Preferably, the said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglyceride in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50 by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackherr under the name Transparent titanium oxide PW®, by the company Miyoshi Kasei under the name UFTR®, by the company Tomen under the name ITS® and by the company Tioxide under the name Tioveil AQ®.

The uncoated zinc oxide pigments are for example:

- those sold under the name Z-Cote by the company Sunsmart;
- those sold under the name Nanox® by the company Elementis;
- those sold under the name Nanogard WCD 2025® by the company Nanophase Technologies.

The coated zinc oxide pigments are for example:

- those sold under the name Zinc Oxide CS-5® by the company Toshibi (ZnO coated with polymethylhydrogenosiloxane);
- those sold under the name Nanogard Zinc Oxide FN® by the company Nanophase Technologies (as a 40% dispersion in Finsolv TN®, C12-C15 alkyl benzoate);
- those sold under the name Daitopersion ZN-30® and Daitopersion ZN-50® by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);

- those sold under the name SPD-Z1® by the company Shin-Etsu (ZnO coated with silicone-grafted acrylic polymer, dispersed in cyclodimethylsiloxane);
- those sold under the name Escalol Z100® by the company ISP (alumina-treated ZnO dispersed in an ethylhexyl methoxycinnamate/PVP-hexadecene copolymer/methicone mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in C12-C15 alkyl benzoate with hydroxystearic acid polycondensate).

The uncoated cerium oxide pigments may be, for example, those sold under the name Colloidal Cerium Oxide® by the company Rhone-Poulenc.

The non-coated iron oxide pigments are sold, for example, by Arnaud under the names Nanogard WCD 2002® (FE 45B®), Nanogard Iron FE 45 BL AQ®, Nanogard FE 45R AQ® and Nanogard WCD 2006® (FE 45R®) or by Mitsubishi under the name TY-220®.

The coated iron oxide pigments are sold, for example, by Arnaud under the names Nanogard WCD 2008 (FE 45B FN)®, Nanogard WCD 2009® (FE 45B 556®), Nanogard FE 45 BL 345® and Nanogard FE 45 BL® or by BASF under the name Transparent Iron Oxide®.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 26® sold by the company Sachtleben Pigments, or coated with alumina, silica and glycerol, such as the product M 211® sold by the company Sachtleben Pigments.

According to the invention, coated or uncoated titanium oxide pigments are particularly preferred.

The additional UV-screening agents according to the invention are preferably present in the compositions according to the invention in a content ranging from 0.1% to 45% by weight and in particular from 5% to 30% by weight relative to the total weight of the composition.

**b) Other additives:**

The compositions in accordance with the present invention may also comprise conventional cosmetic adjuvants chosen in particular from organic solvents, ionic or nonionic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, basifying or acidifying agents or any other ingredient commonly used in the cosmetic and/or dermatological field.

Mention may be made, among organic solvents, of alcohols other than $C_1$-$C_4$ monoalcohols as defined above and in particular short-chain $C_2$-$C_8$ polyols, such as glycerol or diols, such as caprylyl glycol, 1,2-pentanediol, propanediol, butanediol, glycols and glycol ethers, such as ethylene glycol, propylene glycol, butylene glycol, dipropylene glycol or diethylene glycol.

Mention may be made, as thickeners, of carboxyvinyl polymers, such as the Carbopols® (Carbomers) and the Pemulens, such as Pemulen TR1® and Pemulen TR2® (acrylate/$C_{10}$-$C_{30}$ alkyl acrylate copolymer); polyacrylamides, such as, for example, the crosslinked copolymers sold under the names Sepigel 305® (CTFA name: polyacrylamide/$C_{13-14}$ isoparaffin/Laureth 7) or Simulgel 600 (CTFA name: acrylamide/sodium acryloyldimethyl taurate copolymer/isohexadecane/polysorbate 80) by SEPPIC; 2-acrylamido-2-methylpropanesulfonic acid polymers and copolymers, optionally crosslinked and/or neutralized, such as the poly(2-acrylamido-2-methylpropanesulfonic acid) sold by Hoechst under the trade name Hostacerin AMPS® (CTFA name: ammonium polyacryloyldimethyl taurate) or Simulgel 800®, sold by SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, such as Simulgel NS® and Sepinov EMT 10®, sold by SEPPIC; cellulose derivatives, such as hydroxyethylcellulose; polysaccharides and in particular gums, such as xanthan gum; water-soluble or water-dispersible silicone derivatives, such as acrylic silicones, polyether silicones and cationic silicones, and mixtures thereof.

Among the acidifying agents, examples that may be mentioned include mineral or organic acids, for instance hydrochloric acid, orthophosphoric acid, sulfuric acid, carboxylic acids, for instance acetic acid, tartaric acid, citric acid or lactic acid, and sulfonic acids.

Mention may be made, among the basifying agents, by way of example, of ammonia, alkali metal carbonates, alkanolamines, such as mono-, di- and triethanolamines and derivatives thereof, sodium hydroxide or potassium hydroxide.

Preferably, the cosmetic composition comprises one or more basifying agents selected from alkanolamines, in particular triethanolamine, and sodium hydroxide.

In the case of a direct emulsion, the pH of the composition in accordance with the invention is generally between 3 and 12 approximately, preferably between 5 and 11 approximately and more particularly still from 6 to 8.5.

Mention may be made, among the active agents for the care of keratinous substances, such as the skin, lips, scalp, hair, eyelashes or nails, of, for example:

- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or for preventing their decomposition;
- agents for stimulating fibroblast proliferation;
- agents for stimulating keratinocytes proliferation;
- muscle relaxants;
- refreshing agents;
- tensioning agents;
- mattifying agents;
- depigmenting agents;
- propigmenting agents;
- keratolytic agents;
- desquamating agents;
- moisturizers;
- anti-inflammatory agents;
- antimicrobial agents;
- slimming agents;
- agents acting on the energy metabolism of cells;
- insect repellents;
- substance P or CGRP antagonists;
- hair-loss counteractants;
- antiwrinkle agents;
- anti-ageing agents.

A person skilled in the art will choose said active agent(s) according to the desired effect on the skin, hair, eyelashes, eyebrows or nails.

Needless to say, a person skilled in the art will take care to choose the abovementioned optional additional compound or compounds and/or the amounts thereof so that the advantageous properties intrinsically attached to the compositions in accordance with the invention are not, or not substantially, detrimentally affected by the envisaged addition(s).

## GALENICAL FORMS

The compositions according to the invention can be prepared according to the techniques well known to those skilled in the art. They may in particular be in the form of a simple or complex emulsion (O/W, W/O, O/W/O or W/O/W), such as a cream, a milk or a cream gel.

They can also be provided in the anhydrous form, such as, for example, in the form of an oil. The term "anhydrous composition" is understood to mean a composition comprising less than 1% by weight of water, indeed even less than 0.5% of water, and in particular devoid of water, the water not being added during the preparation of the composition but corresponding to the residual water contributed by the mixed ingredients. They can optionally be packaged as an aerosol and be provided in the form of a foam or of a spray.

In the case of compositions in the form of oil-in-water or water-in-oil emulsions, the emulsification processes which can be used are of the paddle or propeller, rotor-stator and HPH type.

In order to obtain stable emulsions with a low content of polymer (oil/polymer ratio > 25), it is possible to prepare

the dispersion in concentrated phase and then to dilute the dispersion with the remainder of the aqueous phase.

It is also possible, via HPH (between 50 and 800 bar), to obtain stable dispersions with droplet sizes that may be as low as 100 nm.

The emulsions generally comprise at least one emulsifier chosen from amphoteric, anionic, cationic and nonionic emulsifiers, used alone or as a mixture. The emulsifiers are appropriately chosen according to the emulsion to be obtained (W/O or O/W).

Mention may be made, as examples of W/O emulsifying surfactants, of alkyl esters or ethers of sorbitan, of glycerol, of polyol or of sugars; or silicone surfactants, such as dimethicone copolyols, for example the mixture of cyclomethicone and dimethicone copolyol sold under the name DC 5225 C® by Dow Corning, and alkyl dimethicone copolyols, such as lauryl methicone copolyol, sold under the name Dow Corning 5200 Formulation Aid by Dow Corning, or cetyl dimethicone copolyol, such as the product sold under the name Abil EM 90R® by Goldschmidt and the mixture of cetyl dimethicone copolyol, polyglycerol (4 mol) isostearate and hexyl laurate sold under the name Abil WE O9® by Goldschmidt. It is also possible to add thereto one or more coemulsifiers which, advantageously, can be chosen from the group consisting of polyol alkyl esters.

Mention may also be made of non-silicone emulsifying surfactants, in particular alkyl esters or ethers of sorbitan, of glycerol, of polyol or of sugars.

Mention may in particular be made, as polyol alkyl esters, of polyethylene glycol esters, such as PEG-30 Dipolyhydroxystearate, such as the product sold under the name Arlacel P135® by ICI.

Mention may be made, as glycerol and/or sorbitan esters, for example, of polyglycerol isostearate, such as the product sold under the name Isolan GI 34® by Goldschmidt; sorbitan isostearate, such as the product sold under the name Arlacel 987® by ICI; sorbitan glyceryl isostearate, such as the product sold under the name Arlacel 986® by ICI, and mixtures thereof.

Mention may be made, for the O/W emulsions, for example, as nonionic emulsifying surfactants, of polyoxyalkylenated (more particularly polyoxyethylenated and/or polyoxypropylenated) esters of fatty acids and of glycerol; oxyalkylenated esters of fatty acids and of sorbitan; polyoxyalkylenated (in particular polyoxyethylenated and/or polyoxypropylenated) esters of fatty acids, optionally in combination with an ester of fatty acid and of glycerol, such as the PEG-100 Stearate/Glyceryl Stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by Henkel under the respective names Plantaren 2000® and Plantaren 1200®, cetearyl glucoside, optionally as a mixture with cetearyl alcohol, sold, for example, under the name Montanov 68® by SEPPIC, under the name Tegocare CG90® by Goldschmidt and under the name Emulgade KE3302® by Henkel, and arachidyl glucoside, for example in the form of the mixture of arachidyl and behenyl alcohols and of arachidyl glucoside sold under the name Montanov 202® by SEPPIC. According to a particular embodiment of the invention, the mixture of the alkyl polyglucoside as defined above with the corresponding fatty alcohol may be in the form of a self-emulsifying composition, for example as described in document WO-A-92/06778.

When it is an emulsion, the aqueous phase of the latter can comprise a nonionic vesicular dispersion prepared according to known processes (Bangham, Standish and Watkins, J. Mol. Biol. 13, 238 (1965), FR 2 315 991 and FR 2 416 008).

The compositions according to the invention find their application in a large number of treatments, especially cosmetic treatments, of the skin, the lips and the hair, including the scalp, especially for protecting and/or caring for the skin, the lips and/or the hair, and/or for making up the skin and/or the lips.

Another subject of the present invention consists of the use of the compositions according to the invention as defined above for the manufacture of products for the cosmetic treatment of the skin, the lips, the nails, the hair, the eyelashes, the eyebrows and/or the scalp, especially care products, antisun products and makeup products.

The cosmetic compositions according to the invention may be used, for example, as makeup products.

Another subject of the present invention consists of a non-therapeutic cosmetic process for caring for and/or making up a keratin material, which consists in applying, to the surface of the said keratin material, at least one composition according to the invention as defined above.

The cosmetic compositions according to the invention may be used, for example, as care products and/or antisun products for the face and/or body with a liquid to semi-liquid consistency, such as milks, more or less smooth creams, cream gels or pastes. They may optionally be packaged as an aerosol and be provided in the form of a foam or of a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged as an aerosol in accordance with the invention generally comprise conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

## ASSEMBLY

According to another aspect, the invention also relates to a cosmetic assembly comprising:

i) a container delimiting one or more compartment(s), the said container being closed by a closing member and optionally being unsealed; and
ii) a make-up and/or care composition in accordance with the invention placed inside the said compartment(s).

The container can, for example, be in the form of a pot or a case.
The closing member can be in the form of a lid comprising a cap mounted so as to be able to move by translation or by pivoting relative to the container housing the said make-up and/or care composition or compositions.
The examples which follow serve to illustrate the invention without, however, exhibiting a limiting nature. In these examples, the amounts of the composition ingredients are given as weight percentages relative to the total weight of the composition.

## Example A1: Preparation of compound (1)

(1)

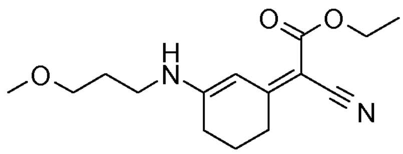

122.23 g of 3-[(3-methoxypropyl)amino]-2-cyclohexen-1-one were alkylated with dimethyl sulfate or alternatively with diethyl sulfate and treated with 75.45 g of ethyl cyanoacetate in approximately equimolar proportions in the presence of a base and optionally of a solvent.
The following base/solvent combinations were used:

| Example | Base | Solvent |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction was monitored, for example, via methods such as TLC, GC or HPLC.
162.30 g of compound (1) were obtained in the form of a brown oil.
After crystallization, the product was obtained in the form of yellowish crystals. Melting point: 92.7°C.

## Example A2: Preparation of compound (2)

(2) 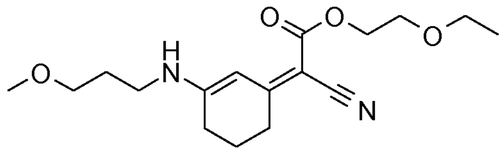

148.4 g of 3-[(3-methoxypropyl)amino]-2-cyclohexen-1-one were alkylated with dimethyl sulfate or alternatively with diethyl sulfate and treated with 130.00 g of 2-ethoxyethyl cyanoacetate in the presence of an organic base and a solvent.

The following base/solvent combinations were used:

| Example | Base | Solvent |
|---|---|---|
| Example A2.1 | DBU (1,8-diazabicyclo[5.4.0]undec-7-ene) | dimethylacetamide |
| Example A2.2 | triethylamine | isopropanol |
| Example A2.3 | 3-methoxypropylamine | isopropanol |
| Example A2.4 | N-methylmorpholine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | No solvent |

**Example A3 (outside the invention): Preparation of the compound (2Z)-2-cyano-N-(3-methoxypropyl)-2-{3-[(3-methoxypropyl)amino]cyclohex-2-en-1-yhdene}ethanamide described in the unpublished patent application PCT/EP 2012/064 195**

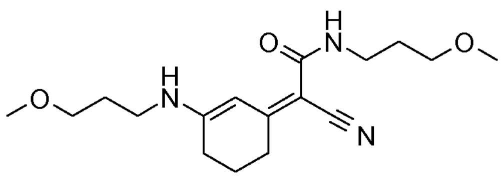

101.00 g of 3-[(3-methoxypropyl)amino]-2-cyclohexen-1-one were alkylated with dimethyl sulfate or alternatively with diethyl sulfate and treated with 86.00 g of 2-cyano-N-(3-methoxypropyl)acetamide in approximately equimolar proportions in the presence of a base and optionally of a solvent.

The following base/solvent combinations were used:

| Example | Base | Solvent |
|---|---|---|
| Example A3.1 | DBU (1,8-diazabicyclo[5.4.0]undec-7-ene) | dimethylacetamide |
| Example A3.2 | triethylamine | isopropanol |
| Example A3.3 | 3-methoxypropylamine | isopropanol |
| Example A3.4 | 3-methoxypropylamine | tert-amyl alcohol |
| Example A3.5 | 3-methoxypropylamine | toluene |
| Example A3.6 | 3-methoxypropylamine | dimethylformamide |
| Example A3.7 | 3-methoxypropylamine | no solvent |

The crude product (2Z)-2-cyano-N-(3-methoxypropyl)-2-{3-[(3-methoxypropyl)amino]cyclohex-2-en-1-ylidene}ethanamide was obtained in the form of a dark brown oil. After chromatography on a column of silica gel (eluent: 99/1 toluene/methanol), 81.8 g of product were obtained in the form of yellowish crystals.

Melting point: 84.7-85.3°C.

## Formulation Examples 1 to 8

The compound 2-ethoxyethyl (2Z)-cyano{3-[(3-methoxypropyl)amino]cyclohex-2-en-1-ylidene}ethanoate (2) of the invention was compared with : the compound (2Z)-2-cyano-N-(3-methoxy-propyl)-2-{3-[(3-methoxypropyl)amino]cyclohex-2-en-1-ylidene}ethanamide according to Example A3 (outside the invention) the compound octyl-5-N,N-diethylamino-2-phenysulfonyl-2,4-pentadienoate (outside the invention) the compound 4-ter-butyl-4'-methoxydibenzoyl methane (PARSOL 1789®) the merocyanine compound MC11 disclosed in the application WO2008/090066 (outside the invention).

Formulations 1 to 4 below were prepared; they were constructed such that the sum of the contents of oil and of liposoluble UV-screening agents remains constant. The content of the screening agents was adjusted so as to ensure the same level of UVB screening and also the same in vitro SPF, and also the same absorbance profile between 290 and 340 nm. For each of the formulations, the SPF, the UVAPPD index and the absorbance after 24 hours at room temperature were measured and at 60°C after 5 days.

| Phase | Ingredients | Formulation 1 (outside the invention) | Formulation 2 (outside the invention) |
|---|---|---|---|
| A | Water | qsp 100 | qsp 100 |
| | Glycerin | 6 | 6 |
| | Disodium EDTA | 0.1 | 0.1 |
| | Triethanolamine | 0.45 | 0.45 |
| | Potassium cetyl phosphate (Amphisol K®) | 1 | 1 |
| B | 2-Ethylphenyl benzoate (X-Tend 226®) | 21.4 | 20.7 |
| | Drometrizole trisiloxane (Mexoryl XL®) | 7.9 | 8.5 |
| | Butyl methoxydibenzoylmethane | 0.7 | - |
| | Octyl 5-N,N-diethylamino-2-phenysulfonyl-2,4-pentad ienoate | - | 0.8 |
| | Stearic acid | 1.5 | 1.5 |
| | Glyceryl Stearate (and) PEG-100 Stearate (Arlacel 165®) | 1.5 | 1.5 |
| | Dimethicone | 0.5 | 0.5 |
| | Preserving agents | 1.28 | 1.28 |
| C | Isohexadecane | 2 | 2 |
| | Xanthan gum | 0.1 | 0.1 |
| | Acrylates / C10-30 Alkyl Acrylate crosspolymer (Pemulen TR1) | 0.25 | 0.25 |
| D | Triethanolamine | 0.25 | 0.25 |
| E | Alcohol | 2 | 2 |
| **in vitro SPF ($t_{24h}$)**<br>**in vitro UVAPPD ($t_{24h}$)** | | 12.4 ± 1.0<br>10.6 ± 0.8 | 12.0 ± 2.2<br>14.4 ± 2.6 |

| Phase | Ingredients | Formulation 3 (outside the invention) | Formulation 4 (invention) |
|---|---|---|---|
| A | Water | qsp 100 | qsp100 |
| | Glycerin | 6 | 6 |
| | Disodium EDTA | 0.1 | 0.1 |
| | Triethanolamine | 0.45 | 0.45 |
| | Potassium Cetyl Phosphate(Amphisol K) | 1 | 1 |
| B | 2-Ethylphenyl benzoate (X-Tend 226®) | 20.5 | 20.1 |
| | Drometrizole Trisiloxane (Mexoryl XL®) | 8.5 | 8.5 |
| | (2Z)-2-cyano-N-(3-methoxypropyl)-2-{3-[(3-methoxypropyl)amino]cyclo-hex-2-en-1-ylidene} ethanamide | 1 | - |
| | Compound (2) | - | 1.4 |
| | Stearic acid | 1.5 | 1.5 |
| | Glyceryl Stearate (and) PEG-100 Stearate (Arlacel 165) | 1.5 | 1.5 |
| | Dimethicone | 0.5 | 0.5 |
| C | Preserving agents | 1.28 | 1.28 |
| | Isohexadecane | 2 | 2 |
| | Xanthan gum | 0.1 | 0.1 |
| | Acrylates / C10-30 Alkyl Acrylate crosspolymer (Pemulen TR1) | 0.25 | 0.25 |
| D | Triethanolamine | 0.25 | 0.25 |
| E | Alcohol | 2 | 2 |
| **in vitro SPF ($t_{24h}$)**<br>**in vitro UVAPPD ($t_{24h}$)** | | 15.4 ± 3.3<br>18.2 ± 3.8 | 14.3 ± 1.4<br>20.0 ± 2.5 |
| **in vitro SPF ($t_{5d}$ at 60°C)**<br>**in vitro UVAPPD ($t_{5d}$ at 60°C)** | | 15.4 ± 1.7<br>16.3 ± 2.2 | 14.4 ± 1.6<br>19.6 ± 2.3 |

The formulations 5 to 8 below were prepared. The content of the filters was constant in order to compare the performance of the compound (2) to the one of the compound MC11 disclosed in the application WO2008/090066 (outside the invention) at the same content. The amounts are expressed in % by weight relative to the total weight of the composition.

| Phase | Ingredients | Formule 5 (invention) | Formule 6 (outside the invention) | Formule 7 (outside the invention) | Formule 8 (outside the invention) |
|---|---|---|---|---|---|
| A | Water | qsp 100 | qsp 100 | qsp 100 | qsp 100 |
| | Glycerin | 5 | 5 | 5 | 5 |
| | Disodium EDTA | 0.1 | 0.1 | 0.1 | 0.1 |
| | Triethanolamine | 0.45 | 0.45 | 0.45 | 0.45 |
| | Potassium Cetyl Phosphate (Amphisol K®) | 1 | 1 | 1 | 1 |

(continued)

| Phase | Ingredients | Formule 5 (invention) | Formule 6 (outside the invention) | Formule 7 (outside the invention) | Formule 8 (outside the invention) |
|---|---|---|---|---|---|
| B | Isopropyl Lauroyl Sarcosinate (Eldew SL-205®) | 30 | 30 | 30 | 30 |
| | Compound (2) | 2 | - | 2 | - |
| | MC11 of WO2008/090066 | - | 2 | - | 2 |
| | Drometrizole Trisiloxane (Mexoryl XL) | 5 | 5 | - | - |
| | Stearic acid | 1.5 | 1.5 | 1.5 | 1.5 |
| | Glyceryl Stearate (and) PEG-100 Stearate (Arlacel 165®) | 2.5 | 2.5 | 2.5 | 2.5 |
| | Dimethicone | 0.5 | 0.5 | 0.5 | 0.5 |
| | Cetyl Alcohol | 0,5 | 0,5 | 0,5 | 0,5 |
| | Cetearyl Alcohol (and) Cetearyl Glucoside (Montanov 68®) | 2 | 2 | 2 | 2 |
| | Preservatives | 1 | 1 | 1 | 1 |
| C | Isohexadecane | 1 | 1 | 1 | 1 |
| | Xanthan Gum | 0.2 | 0.2 | 0.2 | 0.2 |
| | Acrylates / C10-C30 Alkyl Acrylate crosspolymer (Pemulen TR1®) | 0.2 | 0.2 | 0.2 | 0.2 |
| D | Triethanolamine | 0.2 | 0.2 | 0.2 | 0.2 |

**Emulsion preparation method** :

The aqueous phase A and oily phase B were prepared by mixing the starting materials with mechanical stirring at 80°C. Once the aqueous solution A and oily solution B were macroscopically homogeneous, the emulsion was prepared by introducing phase B into phase A with stirring using a rotor-stator homogenizer with a stirring speed of 4500 rpm for 20 minutes. The phases C and then D were then successively added, with continued stirring. The emulsion was finally cooled to room temperature before the addition of phase E when it exists. The final emulsion was characterized by drops of between 1 μm and 20 μm in size.

**In vitro protocol for evaluating the screening efficacy**

The sun protection factor (SPF) was determined according to the in vitro method described by B.L. Diffey in J. Soc. Cosmet. Chem. 40, 127-133 (1989). The measurements were carried out using a UV-1000S spectrophotometer from the company Labsphere. The "static in vitro protection factor (SPF)" value is extracted. Each composition is applied to a rough plate of PMMA in the form of a uniform and even deposit in a proportion of 1 mg/cm2.

The in vitro UVAPPD index measurements were taken under the same conditions using a UV-1000S spectrophotometer from the company Labsphere. The "UVAPPD index (persistent pigment darkening action spectrum)" value is extracted. Each composition is applied to a rough plate of PMMA, in the form of a uniform and even deposit at a rate of 1 mg/cm2.

**Protocol for evaluating the absorbance spectra of the formulations**

The absorbance spectra of the formulations were extracted from the mAF data as a function of the wavelength generated during the in vitro SPF measurement and the in vitro PPD measurement. The mAF values were then converted into absorbance values according to: Abs= log(mAF).

**Absorbance of the formulations measured 24 hours after formulation**

| Absorbance | Formulation 1 (outside the invention) | Formulation 2 (outside the invention) | Formulation 3 (outside the invention) | Formulation 4 (invention) |
|---|---|---|---|---|
| Absorbance at 290 nm after 24 hours | 0.95 ± 0.03 | 0.92 ± 0.07 | 1.04 ± 0.08 | 1.02 ± 0.04 |
| Absorbance at 320 nm after 24 hours | 1.07 ± 0.03 | 1.03 ± 0.08 | 1.15 ± 0.09 | 1.12 ± 0.04 |
| Absorbance at 400 nm after 24 hours | 0.04 ± 0.01 | 0.12 ± 0.01 | 0.53 ± 0.04 | **0.74 ± 0.02** |

**Absorbance of the formulations of formulations 3 and 4 measured after 5 days at 60°C**

| Absorbance | Formulation 3 (outside the invention) | Formulation 4 (invention) |
|---|---|---|
| Absorbance at 290 nm after 5 days at 60°C | 1.01 ± 0.05 | 0.99 ± 0.05 |
| Absorbance at 320 nm after 5 days at 60°C | 1.15 ± 0.05 | 1.12 ± 0.05 |
| Absorbance at 400 nm after 5 days at 60°C | 0.41 ± 0.03 | 0.71 ± 0.05 |

**Conclusions**

The in vitro UVAPPD values showed that, for the same SPF and a similar absorbance profile in UVB and short UVA (from 290 to 340 nm), formulations 1 and 2 are less efficient than formulations 3 and 4.

Formulation 4 according to the invention comprising a lipophilic benzotriazole screening agent and the merocyanine compound (2) has a higher absorbance at 400 nm than formulation 3 comprising the same benzotriazole screening agent and the compound (2Z)-2-cyano-N-(3-methoxypropyl)-2-{3-[(3-methoxypropyl)amino]cyclohex-2-en-1-ylidene}ethanamide. Consequently, formulation 4 of the invention has, relative to formulation 3, a broader absorbance profile in the UV range for comparable in vitro SPF and PPD values. This effect is all the more pronounced after 5 days of storage of the formulations at 60°C.

**Protocol for evaluating the color of the formulations**

The color of the formulations was evaluated after preparation of thin films on contrast map. The formulations were deposited within a circle of 2.2 cm of diameter and planed to obtain thicknesses of reproducible deposit. The colorimetric measures were then made by means of a spectro-colorimeter Minolta CM2600D in two points of the film. This operation is twice reproduced, which leads to 4 experimental values by formulation.

The results are expressed in the system (L*, has*, b*) in which L* represents the luminance, a* represents the red-green axis (-a* = green, +a* = red) and b* represents the yellow-blue axis (-b* blue, +b* yellow). So, a* and b* express the shade of the compound.

The difference of color ΔE* was calculated from the variations L*, a* et b* between the compound (2) and the compound MC11 with the following equation :

$$(\Delta E^*)^2 = (\Delta L^*)^2 + (\Delta a^*)^2 + (\Delta b^*)^2$$

$\Delta L^*$ ± $L^*$ formulation with compound MC11- $L^*$ formulation with compound (2)
$\Delta a^*$ = $a^*$ formulation with compound MC11- $a^*$ formulation with compound (2)
$\Delta b^*$ = $b^*$ formulation with compound MC11 - $b^*$ formulation with compound (2)

We consider that the difference of color between the two compounds is significant if $\Delta E^* > 2$.

**Colorimetric measures on the formulations 5 to 8**

| | **Formulation 5 (invention)** | **Formulation 6 (outside the invention)** | **Formulation 7 (outside the invention)** | **Formulation 8 (outside the invention)** |
|---|---|---|---|---|
| L* | 92.6 ± 0.7 | 92.6 ± 0.7 | 93.1 ± 0.6 | 93.0 ± 0.6 |
| a* | -4.3 ± 0.4 | -6.1 ± 0.2 | -5.08 ± 0.03 | -6.40 ± 0.05 |
| Δa* | -1,8 | | -1,32 | |
| b* | 11.0±1 | 16.8 ± 0.7 | 12.0 ± 0.2 | 15.8 ± 0.2 |
| **Δb*** | **5.8** | | **3.8** | |
| **ΔE*** | **6.1** | | **4.0** | |

The colorimetry results on the examples 5 to 8 show that the formulations 6 and 8 with the compound (2) are significantly less yellow than the equivalent formulations 7 and 9 with the compound MC11 of the application WO2008/090066.

**Formulations 9 to 13**

The compound 2-ethoxyethyl (2Z)-cyano{3-[(3-methoxypropyl)amino]cyclohex-2-en-1-ylidene}ethanoate (2) of the invention was compared with :

the compound (2Z)-2-cyano-N-(3-methoxy-propyl)-2-{3-[(3-methoxypropyl)amino]cyclohex-2-en-1-ylidene}ethanamide according to Example A3 (outside the invention)
the compound octyl-5-N,N-diethylamino-2-phenysulfonyl-2,4-pentadienoate (outside the invention)
the compound 4-ter-butyl- 4'-methoxydibenzoyl methane (PARSOL 1789®)
the merocyanine compound MC11 disclosed in the application WO2008/090066 (outside the invention).

Formulations 9 to 13 below were prepared; they were constructed such that the sum of the contents of oil and of liposoluble UV-screening agents remains constant. The content of the screening agents was adjusted so as to ensure the same level of UVB screening and also the same in vitro SPF, and also the same absorbance profile between 290 and 340 nm. For each of the formulations, the SPF, the UVAPPD index and the absorbance after 24 hours at room temperature.

| Phase | Ingredients | Formulation 9 (outside the invention) | Formulation 10 (outside the invention) |
|---|---|---|---|
| A | Water | qsp100 | qsp100 |
| | Glycerin | 6 | 6 |
| | Disodium EDTA | 0.1 | 0.1 |
| | Triethanolamine | 0.45 | 0.45 |
| | Potassium Cetyl Phosphate (Amphisol K®) | 1 | 1 |
| B | 2-Ethylphenyl benzoate (X-Tend 226®) | 24 | 23,7 |
| | Bis-EthylHexyloxyphenol Methoxyphenyl Triazine (Tinosorb S®) | 4.5 | 5 |
| | Butyl Methoxydibenzoylmethane | 1.5 | - |
| | Octyl-5-N,N-diethylamino-2-phenysulfonyl-2,4-pentadienoate | - | 1.3 |
| | Stearic Acid | 1.5 | 1.5 |
| | Glyceryl Stearate (and) PEG-100 Stearate (Arlacel 165®) | 1.5 | 1.5 |
| | Dimethicone | 0.5 | 0.5 |
| | Preservatives | 1.28 | 1.28 |
| C | Isohexadecane | 2 | 2 |
| | Xanthan gum | 0.1 | 0.1 |
| | Acrylates / C10-C30 Alkyl Acrylate crosspolymer (Pemulen TR1®) | 0.25 | 0.25 |
| D | Triethanolamine | 0.25 | 0.25 |
| E | Alcohol | 2 | 2 |
| **SPF in vitro at $t_{24h}$**<br>**UVA PPD in vitro at $t_{24h}$** | | 13.0 ± 1.1<br>13.2 ± 1.0 | 13.0 ± 1.9<br>17.6 ± 2.9 |

| Phase | Ingredients | Formulation 11 (outside the invention) | Formulation 12 (invention) |
|---|---|---|---|
| A | Water | qsp 100 | qsp 100 |
| | Glycerin | 6 | 6 |
| | Disodium EDTA | 0.1 | 0.1 |
| | Triethanolamine | 0.45 | 0.45 |
| | Potassium Cetyl Phosphate (Amphisol K®) | 1 | 1 |

(continued)

| Phase | Ingredients | Formulation 11 (outside the invention) | Formulation 12 (invention) |
|---|---|---|---|
| B | 2-Ethylphenyl benzoate (X-Tend 226®) | 23.3 | 23 |
| | Bis-Ethyl Hexyloxyphenol Methoxyphenyl Triazine (Tinosorb S®) | 5 | 5 |
| | (2Z)-2-cyano-N-(3-methoxypropyl)-2-{3-[(3-methoxypropyl)amino]cyclohex-2-en-1-ylidene} ethanamide | 1.7 | - |
| | Compound (2) | - | 2 |
| | Stearic Acid | 1.5 | 1.5 |
| | Glyceryl Stearate (and) PEG-100 Stearate (Arlacel 165®) | 1.5 | 1.5 |
| | Dimethicone | 0.5 | 0.5 |
| | Preservatives | 1.28 | 1.28 |
| C | Isohexadecane | 2 | 2 |
| | Xanthan gum | 0.1 | 0.1 |
| | Acrylates / C10-30 Alkyl Acrylate crosspolymer (Pemulen TR1®) | 0.25 | 0.25 |
| D | Triethanolamine | 0.25 | 0.5 |
| E | Alcohol | 2 | 2 |
| **SPF in vitro at $t_{24h}$**<br>**UVA PPD in vitro at $t_{24h}$** | | 12.4 ± 2.3<br>19.3 ± 4.9 | 16.7 ± 3.8<br>23.6 ± 5.7 |

The formulations 12 and 13 below were prepared. The content of the filters was constant in order to compare the perfomance of the compound (2) according to the invention to the one of compound MC11 disclosed in the application WO2008/090066 (outside the invention) at the same content. The amounts are expressed in % by weight relative to the total weight of the composition.

| Phase | Ingredients | Formulation 12 (invention) | Formulation 13 (outside the inven tion) |
|---|---|---|---|
| A | Water | qsp 100 | qsp 100 |
| | Glycerin | 6 | 6 |
| | Disodium EDTA | 0.1 | 0.1 |
| | Triethanolamine | 0.45 | 0.45 |
| | Potassium Cetyl Phosphate (Amphisol K®) | 1 | 1 |

(continued)

| Phase | Ingredients | Formulation 12 (invention) | Formulation 13 (outside the inven tion) |
|---|---|---|---|
| B | 2-Ethylphenyl benzoate (X-Tend 226®) | 23 | 23 |
| | Bis-Ethyl Hexyloxyphenol Methoxyphenyl Triazine (Tinosorb S®) | 5 | 5 |
| | Compound (2) | 2 | - |
| | MC11 of WO2008/090066 | - | 2 |
| | Stearic Acid | 1.5 | 1.5 |
| | Glyceryl Stearate (and) PEG-100 Stearate (Arlacel 165®) | 1.5 | 1.5 |
| | Dimethicone | 0,5 | 0,5 |
| | Preservatives | 1,28 | 1,28 |
| C | Isohexadecane | 2 | 2 |
| | Xanthan gum | 0,1 | 0,1 |
| | Acrylates / C10-30 Alkyl Acrylate crosspolymer (Pemulen TR1®) | 0.25 | 0.25 |
| D | Triethanolamine | 0.25 | 0.25 |
| E | Alcohol | 2 | 2 |

The emulsions 9 to 13 were prepared according to the same mode of preparation of the examples 1 to 8.

The SPF and UVAPPD in vitro indexes and the absorbance profiles measured after 24 hours were measured in the same conditions as previously indicated.

**<u>Absorbance of the formulations 9 to 12 measured 24 hours after formulation</u>**

| Absorbance | Formulation 9 (outside the invention) | Formulation 10 (outside the invention) | Formulation 11 (outside the invention) | Formulation 12 (invention) |
|---|---|---|---|---|
| Absorbance at 290 nm (t24h) | 0.88 ± 0.03 | 0.86 ± 0.05 | 0.83 ± 0.07 | 0.97 ± 0.09 |
| Absorbance at 320 nm (t24h) | 1.16 ± 0.04 | 1.13 ± 0.07 | 1.11 ± 0.08 | 1.23 ± 0.11 |
| Absorbance at 400 nm (t24h) | 0.07 ± 0,01 | 0.17 ± 0.01 | 0.66 ± 0.03 | 0.87 ± 0.08 |

The in vitro UVAPPD values showed that, for the same SPF and a similar absorbance profile in UVB and short UVA (from 290 to 340 nm), the formulation 9 is less efficient than the formulations 10, 11 and 12.

Formulation 12 according to the invention comprising a bis-resorcinyl triazine compound and the merocyanine compound (2) has a higher absorbance at 400 nm than formulations 10 and 11 comprising the same bis-résorcinyl triazine and respectively the compound octyl-5-N,N-diethylamino-2-phenysulfonyl-2,4-pentadienoate or the compound (2Z)-2-cyano-N-(3-methoxypropyl)-2-{3-[(3-methoxypropyl)-amino]-cyclohex-2-en-1-ylidene}-ethanamide. Consequently, formulation 12 of the invention has, relative to formulations 9, 10 and 11, a broader absorbance profile in the UV range for comparable in vitro SPF and PPD values.

## Colorimetric measurements on the formulations 12 and 13

The colorimetric measurements on the formulations 12 and 13 were made in the same conditions as previously indicated.

| | Formulation 12 (invention) | Formulation 13 (outside the invention) |
|---|---|---|
| L* | 93.8 ± 0.6 | 9.6 ± 0.5 |
| a* | -6.4 ± 0.1 | -7.8 ± 0.1 |
| Δa* | -1,4 | |
| b* | 16.5 ± 0,2 | 20.5 ± 0.4 |
| Δ**b*** | **4** | |
| Δ**E*** | **4.3** | |

The colorimetry results on the examples 12 and 13 show that the formulation 12 with the compound (2) is significantly less yellow than the equivalent formulation 13 with the compound MC11 of the application WO2008/ 090066.

## Claims

**1.** Cosmetic or dermatological composition comprising, in a physiologically acceptable support:

a) at least one oily phase and
b) at least one merocyanine compound corresponding to formula (I) below, and also the E/E- or E/Z- geometrical isomer forms thereof:

(I)

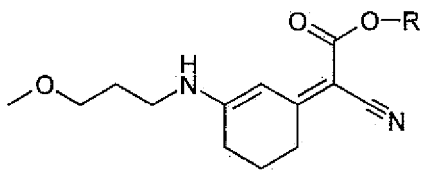

in which:
R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being substituted with one or more O, and
c) at least one lipophilic benzotriazole UV-screening agent and/or one bis-resorcinyl triazine compound;
the said composition containing less than 2% of cyclohexasiloxane relative to the total weight of the composition when it contains at least one lipophilic benzotriazole UV-screening agent, excluding the following O/W emulsion in which the amounts of the ingredients are expressed as percentage of weight relative to the total weight of the composition:

| Ingredients | %wt |
|---|---|
| Caprylyl glycol | 0.5 |
| Caprylic/Capric Triglyceride and Sodium Acrylates Copolymer (Luvigel EM -BASF) | 3 |
| Triethanolamine | 0.2 |
| Ethylhexyl salicylate | 5 |
| Drometrizole trisiloxane | 2 |
| Inulin Lauryl Carbamate (Inutec SP1 - ORAFTI) | 0.3 |
| Cyclohexasiloxane | 2 |

(continued)

| Ingredients | %wt |
|---|---|
| Glycerin | 5 |
| Propylene glycol | 10 |
| $C_{12}$-$C_{15}$-Alkyl Benzoate (Finsolv TN - INNOSPEC ACTIVE CHEMICAL) | 7 |
| Octocrylene | 7 |
| Butyl methoxydibenzoylmethane | 3 |
| **ethyl(2Z)-cyano{3-[(3-methoxypropyl)amino]cyclohex-2-en-1-ylidene}ethanoate** | **3** |
| Terephtalylidene Dicamphor Sulfonic acid | 0.5 |
| Preservative | 0.8 |
| Disodium EDTA | 0.1 |
| Water | qs100 |

2. Composition according to Claim 1, in which the merocyanine compound(s) of formula (I) are chosen from those in which:
R is a $C_1$-$C_{22}$ alkyl, which may be substituted with one or more O.

3. Composition according to Claim 1 or 2, in which the merocyanine compound(s) of formula (I) are chosen from the following compounds, and also the E/E- or E/Z-geometrical isomer forms thereof:

| | | | |
|---|---|---|---|
| 1 | 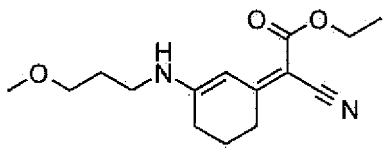 ethyl (2Z)-cyano{3-[(3-methoxypropyl)amino] cyclohex-2-en-1-ylidene}ethanoate | 4 | 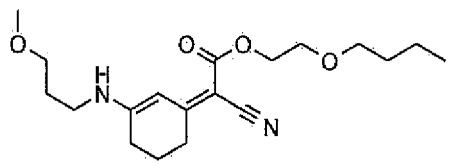 2-butoxyethyl (2Z)-cyano{3-[(3-methoxypropyl) amino]cyclohex-2-en-1-ylidene}ethanoate |
| 2 | 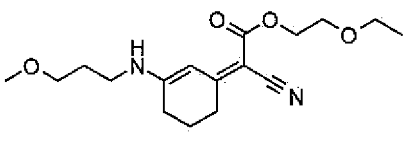 2-ethoxyethyl (2Z)-cyano{3-[(3-methoxypropyl) amino]cyclohex-2-en-1-ylidene}ethanoate | 5 | 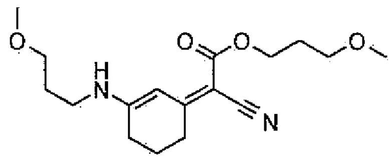 3-methoxypropyl (2Z)-cyano{3-[(3-methoxypropyl) amino]cyclohex-2-en-1-ylidene}ethanoate |
| 3 | 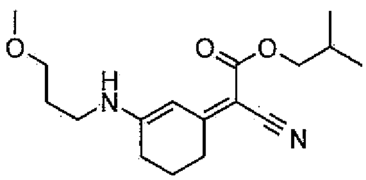 2-methylpropyl (2Z)-cyano{3-[(3-methoxypropyl) amino]cyclohex-2-en-1-ylidene}ethanoate | 6 | 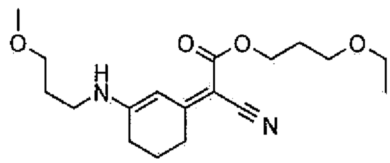 3-ethoxypropyl (2Z)-cyano{3-[(3-methoxypropyl) amino]cyclohex-2-en-1-ylidene}ethanoate |

4. Composition according to Claim 3, in which the merocyanine compound(s) of formula (I) are chosen from the following compounds, and also the E/E- or E/Z-geometrical isomer forms thereof:

| | | | |
|---|---|---|---|
| 2 | 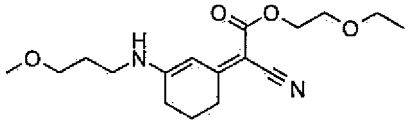<br>2-ethoxyethyl (2Z)-cyano{3-[(3-methoxypropyl) amino]cyclohex-2-en-1-ylidene}ethanoate | 5 | 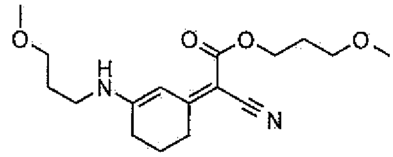<br>3-methoxypropyl (2Z)-cyano{3-[(3-methoxypropyl) amino]cyclohex-2-en-1-ylidene}ethanoate |
| 3 | 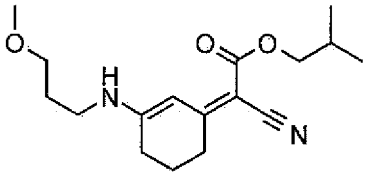<br>2-methylpropyl {2Z)-cyano{3-[(3-methoxypropyl) amino]cyclohex-2-en-1-ylidene}ethanoate | 6 | 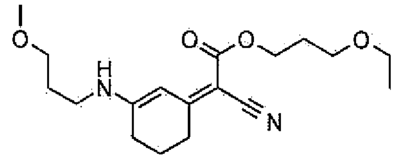<br>3-ethoxypropyl (2Z)-cyano{3-[(3-methoxypropyl) amino]cyclohex-2-en-1-ylidene}ethanoate |
| 4 | 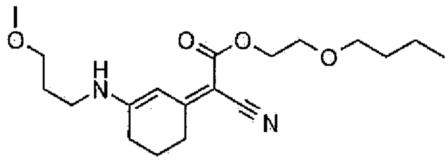<br>2-butoxyethyl (2Z)-cyano{3-[(3-methoxypropyl) amino]cyclohex-2-en-1-ylidene}ethanoate | | |

**5.** Composition according to Claim 4, in which the merocyanine compound is 2-ethoxyethyl (2Z)-cyano{3-[(3-methoxypropyl)amino]cyclohex-2-en-1-ylidene}ethanoate (2) in its E/Z geometrical configuration having the following structure:

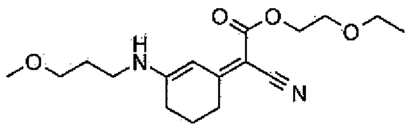

and/or in its E/E geometrical configuration having the following structure:

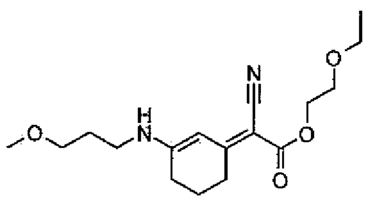

**6.** Composition according to any one of Claims 1 to 5, in which the merocyanine compound(s) of formula (I) are present in a concentration ranging from 0.1% to 10% by weight and preferentially from 0.2% to 5% by weight relative to the total weight of the composition.

**7.** Composition according to any one of Claims 1 to 6, in which the lipophilic benzotriazole screening agent is chosen from:

- 2-benzotriazolyl-4-tert-octyl phenol,
- 2-(2H-benzotriazol-2-yl)-4-methylphenol,
- 2-2(H-benzotriazol-2-yl)-p-cresol,

- 2-(2'-hydroxy-3'-butyl-5'-methylphenyl)benzotriazole,
- 2-(2'-hydroxy-5'-t-octylphenyl)benzotriazole,
- benzotriazolyl dodecyl p-cresol,
- 2-t-butyl-6-(5-chloro-2H-benzotriazol-2-yl)-p-cresol,
- the benzotriazole compounds of formula

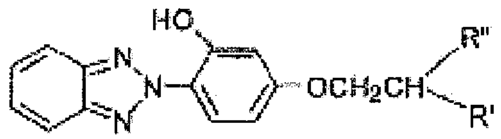

in which R' denotes a linear $C_1$-$C_6$ alkyl and R" denotes a $C_1$-$C_3$ alkyl, in particular 2-[4-(2-ethylhexyloxy)-2-hydroxyphenyl]-2H-benzotriazole and 2-(2-hydroxy-4-isobutoxyphenyl)-2H-benzotriazole,
- and mixtures thereof.

**8.** Composition according to any one of Claims 1 to 6, in which the lipophilic benzotriazole UV-screening agent is chosen from silanes or siloxanes bearing a benzotriazole function comprising at least one unit of formula (1) below:

$$O_{(3-a)/2}Si(R_7)_a\text{-}G \qquad (1)$$

in which:

- $R_7$ represents an optionally halogenated $C_1$-$C_{10}$ alkyl radical or a phenyl radical or a trimethylsiloxy radical,
- a is an integer chosen between 0 and 3 inclusive,
- and the symbol G denotes a monovalent radical directly bonded to a silicon atom, and which corresponds to formula (2) below:

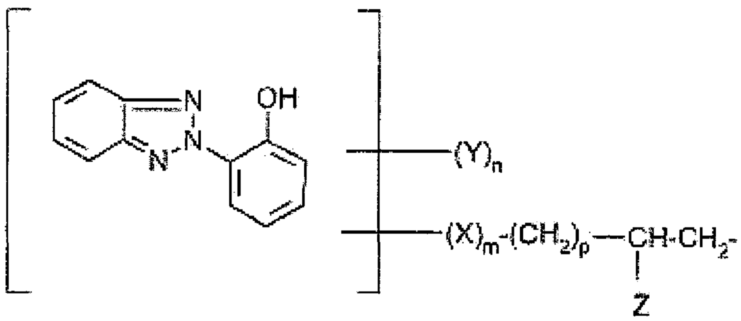

(2)

in which:

- Y, which may be identical or different, are chosen from $C_1$-$C_8$ alkyl radicals, halogens and $C_1$-$C_4$ alkoxy radicals, it being understood that, in the latter case, two adjacent Y of the same aromatic nucleus may together form an alkylidenedioxy group in which the alkylidene group contains from 1 to 2 carbon atoms,
- X represents O or NH,
- Z represents hydrogen or a $C_1$-$C_4$ alkyl radical,
- n is an integer between 0 and 3 inclusive,
- m is 0 or 1,
- p represents an integer between 1 and 10 inclusive.

**9.** Composition according to claim 8, in which the lipophilic benzotriazole UV-screening agent corresponds to formula (5) or (6) below:

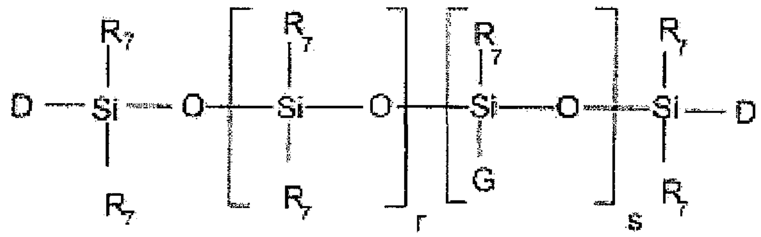

(5)

or

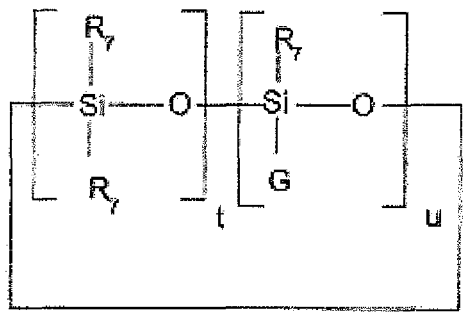

(6)

in which:

- $R_7$, which may be identical or different, are chosen from $C_1$-$C_{10}$ alkyl, phenyl, 3,3,3-trifluoropropyl and trimethylsiloxy radicals, at least 80% by number of the radicals $R_7$ being methyl,
- D, which may be identical or different, are chosen from the radicals $R_7$ and the radical G,
- r is an integer between 0 and 50 inclusive, and s is an integer between 0 and 20 inclusive, and if s = 0, at least one of the two symbols D denotes G,
- u is an integer between 1 and 6 inclusive, and t is an integer between 0 and 10 inclusive, it being understood that t + u is equal to or greater than 3,
- and the symbol G corresponds to formula (2) above.

**10.** Composition according to Claim 9, in which the benzotriazole UV-screening agent is chosen from those of formula (5), in particular from those for which the radicals D are both radicals $R_7$.

**11.** Composition according to Claim 9 or 10, in which the benzotriazole UV-screening agent of formula (5) has at least one and even more preferentially all of the following characteristics:

- D is a radical $R_7$,
- $R_7$ is alkyl and even more preferentially is methyl,
- r is between 0 and 15 inclusive; s is between 1 and 10 inclusive,
- n is non-zero and preferably equal to 1, and Y is then chosen from methyl, tert-butyl and $C_1$-$C_4$ alkoxy,
- Z is hydrogen or methyl,
- m = 0, or [m = 1 and X =O],
- p is equal to 1.

**12.** Composition according to either of Claims 9 to 11, in which the lipophilic benzotriazole UV-screening agent corresponds to the general formula (7) below:

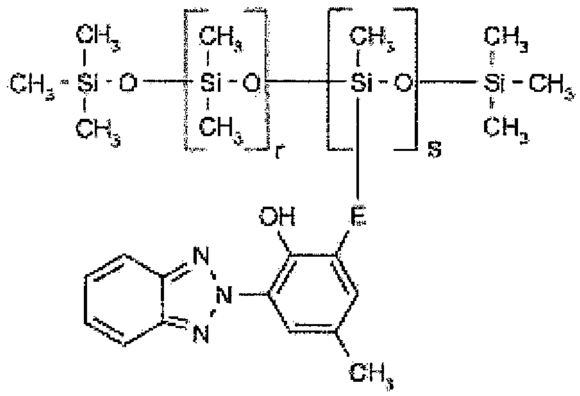

(7)

with $0 \leq r \leq 10$,
$1 \leq s \leq 10$,
and in which E represents the divalent radical:

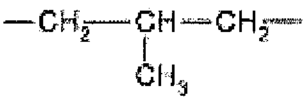

**13.** Composition according to claim 12, in which the benzotriazole UV-screening agent of formula (7) is the compound corresponding to the following formula:

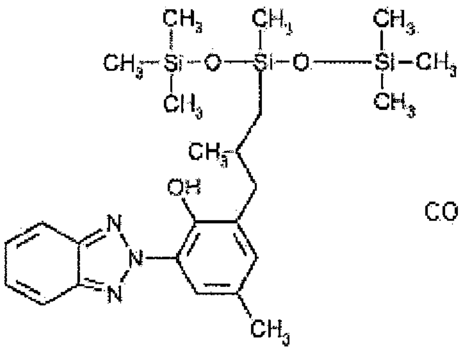

compound (a)

**14.** Composition according to any one of Claims 1 to 6, in which the bis-resorcinyl triazine compound corresponds to formula (II) below:

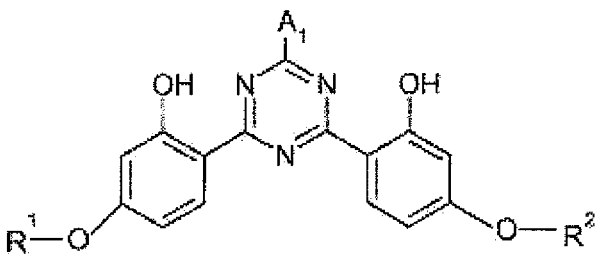

(II)

in which:

(i) the radicals $R^1$ and $R^2$, which may be identical or different, denote a $C_3$-$C_{18}$ alkyl radical; a $C_2$-$C_{18}$ alkenyl radical or a residue of formula $-CH_2-CH(OH)-CH_2-OT_1$ in which $T_1$ is a hydrogen atom or a $C_1$-$C_8$ alkyl radical; or
(ii) the radicals $R^1$ and $R^2$, which may be identical or different, denote a residue of formula (III) below:

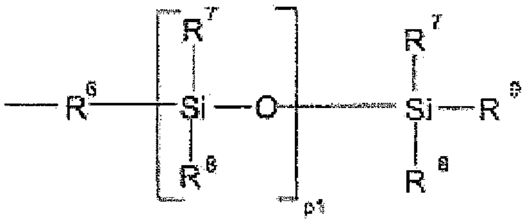

(III)

in which:

- $R^6$ denotes a covalent bond; a linear or branched $C_1$-$C_4$ alkylene radical or a residue of formula $-C_{m1}H_{2m1}-$ or $-C_{m1}H_{2m1}-O-$ in which m1 is a number from 1 to 4;
- p1 is a number from 0 to 5;
- the radicals $R^7$, $R^8$ and $R^9$, which may be identical or different, denote a $C_1$-$C_{18}$ alkyl radical; a $C_1$-$C_{18}$ alkoxy radical or a residue of formula:

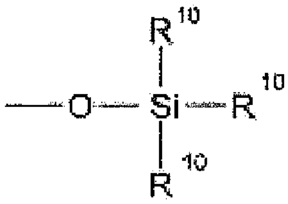

(IV)

in which $R^{10}$ is a $C_1$-$C_5$ alkyl radical;
- $A_1$ denotes a residue corresponding to one of the following formulae:

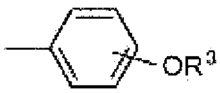

(V)

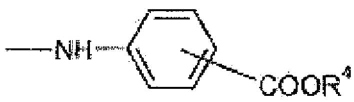

(VI)

10

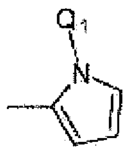

(VII)

in which:

- $R^3$ denotes a hydrogen atom, a $C_1$-$C_{10}$ alkyl radical or a radical of formula: $-(CH_2CHR^5-O)_{n1}$ $R^4$ in which n1 is a number from 1 to 16,
- $R^4$ denotes hydrogen, a metal cation M, a $C_1$-$C_5$ alkyl radical or a residue of formula $-(CH_2)_{m2}-OT_1$ in which m2 is a number from 1 to 4 and $T_1$ has the same meaning indicated in paragraph (i),
- $R^5$ is hydrogen or methyl, or a residue of structure $=CH_2-CH-(OH)-CH_2OT_1$ with $T_1$ having the same meaning indicated in paragraph (i),
- $Q_1$ is a $C_1$-$C_{18}$ alkyl radical.

**15.** Composition according to Claim 14, in which the bis-resorcinyl triazine compound of formula (II) is chosen from:

2,4-bis{[4-(3-(2-propyloxy)-2-hydroxypropyloxy)-2-hydroxy]phenyl}-6-(4-methoxyphenyl)-1,3,5-triazine;
- 2,4-bis{[4-(2-ethylhexyloxy)-2-hydroxy]phenyl}-6-[4-(2-methoxyethyl-carboxyl)phenylamino]-1,3,5-triazine;
- 2,4-bis{[4-tris(trimethylsiloxysilylpropyloxy)-2-hydroxy]phenyl}-6-(4-methoxyphenyl)-1,3,5-triazine;

- 2,4-bis{[4-(2''-methylpropenyloxy)-2-hydroxy]phenyl}-6-(4-methoxyphenyl)-1,3,5-triazine;
- 2,4-bis{[4-(1',1',1',3',5',5',5'-heptamethy)trisiloxy-2''-methylpropyloxy)-2-hydroxy]phenyl}-6-(4-methoxyphenyl)-1,3,5-triazine,
2,4-bis{[4-(3-(2-propyloxy)-2-hydroxypropyloxy]-2-hydroxy]phenyl}-6-[(4-ethylcarboxy)phenylamino]-1,3,5-triazine;
- 2,4-bis{[4-(2-ethylhexyloxy)-2-hydroxy]phenyl}-6-(1-methyl-2-pyrrolyl)-1,3,5-triazine, and mixtures thereof, and even more particularly the bis-resorcinyl triazine compound of formula (II) is 2,4-bis{[4-(2-ethylhexyloxy)-2-hydroxy]phenyl}-6-(4-methoxyphenyl)-1,3,5-triazine.

**16.** Composition as defined in any one of the preceding claims for use in a method for caring for and/or making up a keratin material, comprising the application to the surface of the said keratin material of the said composition .

**17.** Composition as defined in any one of the preceding claims for use in a method for limiting the darkening of the skin and/or improving the colour and/or the uniformity of the complexion, comprising the application to the surface of the skin of the said composition .

**18.** Composition as defined in any one of the preceding claims for use in a method for preventing and/or treating the signs of ageing of a keratin material, comprising the application to the surface of the keratin material of the said composition.

**Patentansprüche**

**1.** Kosmetische oder dermatologische Zusammensetzung, umfassend in einem physiologisch unbedenklichen Träger:

a) mindestens eine ölige Phase und
b) mindestens eine Merocyanin-Verbindung der nachstehenden Formel (I) sowie die geometrischen E/E- oder E/Z-Isomerformen davon:

(I)

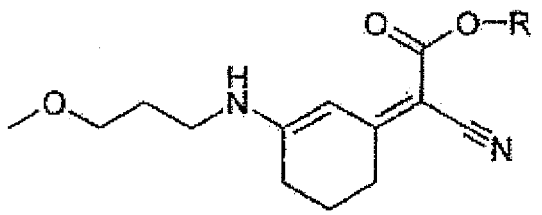

wobei:
R für eine $C_1$-$C_{22}$-Alkylgruppe, eine $C_2$-$C_{22}$-Alkenylgruppe, eine $C_2$-$C_{22}$-Alkinylgruppe, eine $C_3$-$C_{22}$-Cycloalkylgruppe oder eine $C_3$-$C_{22}$-Cycloalkenyl-gruppe steht, wobei die Gruppen gegebenenfalls durch ein oder mehrere O substituiert sein können, und
c) mindestens eine lipophile Benzotriazol-UV-Filtersubstanz und/oder eine Bisresorcinyltriazin-Verbindung;
wobei die Zusammensetzung weniger als 2 % Cyclohexasiloxan, bezogen auf das Gesamtgewicht der Zusammensetzung, enthält, wenn sie mindestens eine lipophile Benzotriazol-UV-Filtersubstanz enthält, unter Ausschluss der folgenden O/W-Emulsion, in der die Mengen der Bestandteile in Gewichtsprozent, bezogen auf das Gesamtgewicht der Zusammensetzung, ausgedrückt sind:

| **Bestandteile** | **Gew.-%** |
|---|---|
| Caprylylglykol | 0,5 |
| Caprylic/Capric Triglyceride und Sodium Acrylates Copolymer (Luvigel EM - BASF) | 3 |
| Triethanolamin | 0, 2 |
| Ethylhexylsalicylat | 5 |
| Drometrizoltrisiloxan | 2 |

(fortgesetzt)

| Bestandteile | Gew.-% |
|---|---|
| Inulin Lauryl Carbamate (Inutec SP1 - ORAFTI) | 0,3 |
| Cyclohexasiloxan | 2 |
| Glycerin | 5 |
| Propylenglykol | 10 |
| $C_{12}$-$C_{15}$-Alkyl Benzoate (Finsolv TN - INNOSPEC ACTIVE CHEMICAL) | 7 |
| Octocrylen | 7 |
| Butylmethoxydibenzoylmethan | 3 |
| **(2Z)-Cyano{3-[(3-methoxy propyl)amino]cyclohex-2-en- 1-yliden}ethansäureethyl- ester** | **3** |
| Terephthalylidene Dicamphor Sulfonic Acid | 0,5 |
| Konservierungsmittel | 0,8 |
| Dinatrium-EDTA | 0,1 |
| Wasser | q.s. 100 |

2. Zusammensetzung nach Anspruch 1, wobei die Merocyanin-Verbindung(en) der Formel (I) aus denjenigen ausgewählt sind, in denen:
R für ein $C_1$-$C_{22}$-Alkyl, das durch ein oder mehrere O substituiert sein kann, steht.

3. Zusammensetzung nach Anspruch 1 oder 2, wobei die Merocyanin-Verbindung(en) der Formel (I) aus den folgenden Verbindungen sowie den geometrischen E/E- oder E/Z-Isomerformen davon ausgewählt sind:

| | | | |
|---|---|---|---|
| 1 | 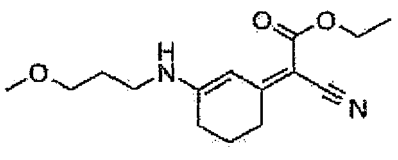 (2Z)-Cyano{3-[(3-methoxypropyl) amino]-cyclohex-2-en-1-yliden}ethansäure-ethylester | 4 | 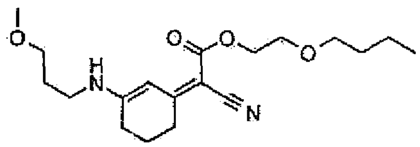 (2Z)-Cyano{3-[(3-methoxypropyl)amino]-cyclohex-2-en-1-yliden}ethansäure-2-butoxyethylester |
| 2 | 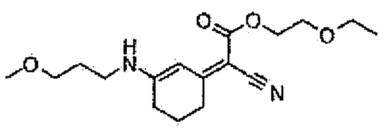 (2Z)-Cyano{3-[(3-methoxypropyl) amino]-cyclohex-2-en-1-yliden}ethansäure-2-ethoxyethylester | 5 | 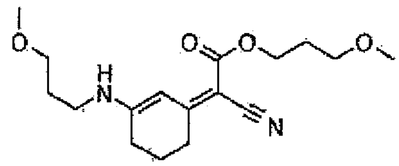 (2Z)Cyano{3-[(3-methoxypropyl)amino]-cyclohex-2-en-1-yliden}ethansäure-3-methoxypropylester |
| 3 | 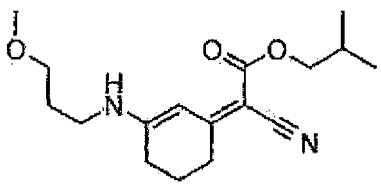 (2Z)-Cyano{3-[(3-methoxypropyl) amino]-cyclohex-2-en-1-yliden}ethansäure-2-methylpropylester | 6 | 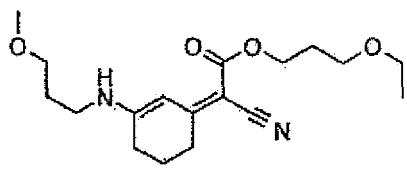 (2Z)-Cyano{3-[(3-methoxypropyl)amino]-cyclohex-2-en-1-yliden}ethansäure-3-ethoxypropylester |

**4.** Zusammensetzung nach Anspruch 3, wobei die Merocyanin-Verbindung(en) der Formel (I) aus den folgenden Verbindungen sowie den geometrischen E/E- oder E/Z-Isomerformen davon ausgewählt sind:

| | | | |
|---|---|---|---|
| 2 | 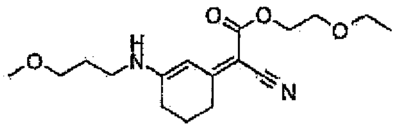<br>(2Z)-Cyano{3-[(3-methoxypropyl)amino]-cyclohex-2-en-1-yliden}ethansäure-2-ethoxyethylester | 5 | 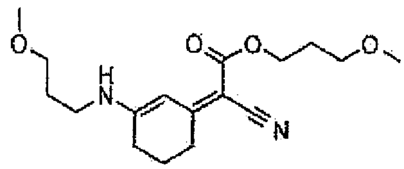<br>(2Z)-Cyano{3-[{3-methoxypropyl)amino]-cyclohex-2-en-1-yliden}ethansäure-3-methoxypropylester |
| 3 | 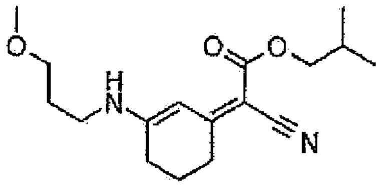<br>(2Z)-Cyano{3-[(3-methoxypropyl)amino]-cyclohex-2-en-1-yliden}ethansäure-2-methylpropylester | 6 | 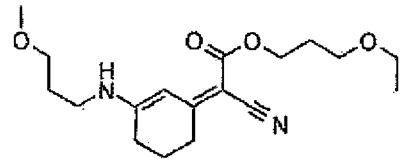<br>(2Z)-Cyano{3-[(3-methoxypropyl)amino]-cyclohex-2-en-1-yliden}ethansäure-3-ethoxypropylester |
| 4 | 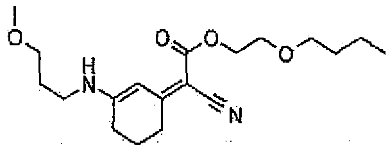<br>(2Z)-Cyano{3-[(3-methoxypropyl)amino]-cyclohex-2-en-1-yliden}ethansäure-2-butoxyethylester | | |

**5.** Zusammensetzung nach Anspruch 4, wobei es sich bei der Merocyanin-Verbindung um (2Z)-Cyano{3-[(3-methoxypropyl)amino] cyclohex-2-en-1-yliden}ethansäure-2-ethoxyethylester (2) in seiner geometrischen E/Z-Konfiguration mit der folgenden Struktur:

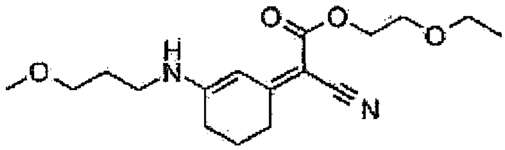

und/oder in seiner geometrischen E/E-Konfiguration mit der folgenden Struktur:

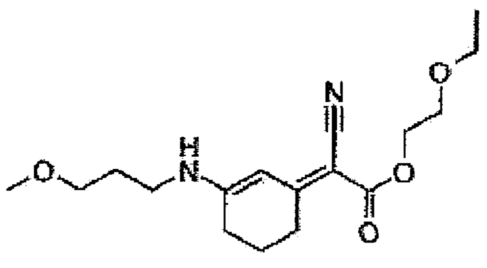

handelt.

**6.** Zusammensetzung nach einem der Ansprüche 1 bis 5, wobei die Merocyanin-Verbindung(en) der Formel (I) in einer Konzentration im Bereich von 0,1 bis 10 Gew.-% und vorzugsweise von 0,2 bis 5 Gew.-%, bezogen auf das Gesamtgewicht der Zusammensetzung, vorliegt bzw. vorliegen.

7. Zusammensetzung nach einem der Ansprüche 1 bis 6, wobei die lipophile Benzotriazol-Filtersubstanz aus:

- 2-Benzotriazolyl-4-tert-octylphenol,
- 2-(2H-Benzotriazol-2-yl)-4-methylphenol,
- 2-(2H-Benzotriazol-2-yl)-p-kresol,
- 2-(2'-Hydroxy-3'-butyl-5'-methylphenyl)benzotriazol,
- 2-(2'-Hydroxy-5'-t-octylphenyl)benzotriazol,
- Benzotriazolyldodecyl-p-kresol,
- 2-t-Butyl-6-(5-chlor-2H-benzotriazol-2-yl)-p-kresol,
- den Benzotriazol-Verbindungen der Formel

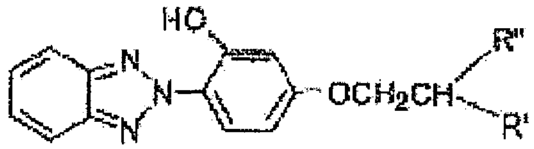

wobei R' für ein lineares $C_1$-$C_6$-Alkyl steht und R" für ein $C_1$-$C_3$-Alkyl steht, insbesondere 2-[4-(2-Ethylhexyloxy)-2-hydroxyphenyl]-2H-benzotriazöl und 2-(2-Hydroxy-4-isobutoxyphenyl)-2H-benzotriazol
- und Mischungen davon

ausgewählt ist.

8. Zusammensetzung nach einem der Ansprüche 1 bis 6, bei dem die lipophile Benzötriazol-UV-Filtersubstanz aus Silanen oder Siloxanen mit einer Benzotriazol-Funktion, die mindestens eine Einheit der nachstehenden Formel (1) umfassen, ausgewählt ist:

$$O_{(3-a)/2}Si(R_7)_a\text{-}G \qquad (1)$$

wobei:

- $R_7$ für einen gegebenenfalls halogenierten $C_1$-$C_{10}$-Alkylrest oder einen Phenylrest oder einen Trimethylsiloxyrest steht,
- a für eine ganze Zahl steht, die zwischen 0 und 3 inklusive ausgewählt ist,
- und das Symbol G für einen einwertigen Rest, der direkt an ein Siliciumatom gebunden ist und der der nachstehenden Formel (2) entspricht, steht:

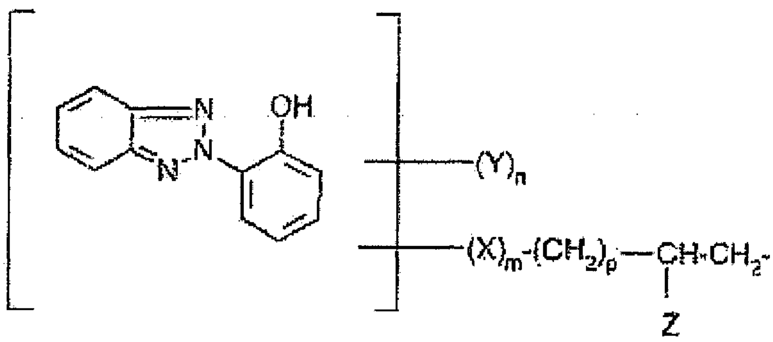

(2)

wobei:

- die Variablen Y, die gleich oder verschieden sein können, aus $C_1$-$C_8$-Alkylresten, Halogen und $C_1$-$C_4$-Alkoxyresten ausgewählt sind, wobei es sich versteht, dass in letzterem Fall zwei benachbarte Variablen Y desselben aromatischen Kerns zusammen eine Alkylidendioxygruppe bilden können, wobei die Alkylidengruppe 1 bis 2 Kohlenstoffatome enthält,
- X für O oder NH steht,
- Z für Wasserstoff oder einen $C_1$-$C_4$-Alkylrest steht,
- n für eine ganze Zahl zwischen 0 und 3 inklusive steht,

- m für 0 oder 1 steht,
- p für eine ganze Zahl zwischen 1 und 10 inklusive steht.

**9.** Zusammensetzung nach Anspruch 8, wobei die lipophile Benzotriazol-UV-Filtersubstanz der nachstehenden Formel (5) oder (6) entspricht:

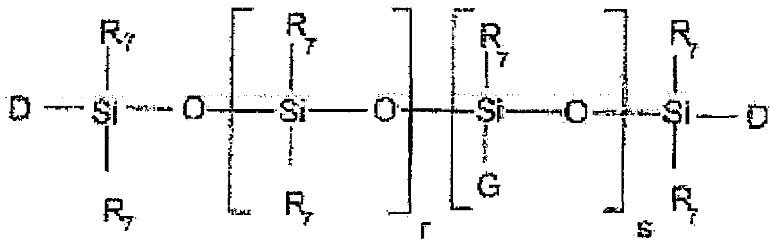

(5)

oder

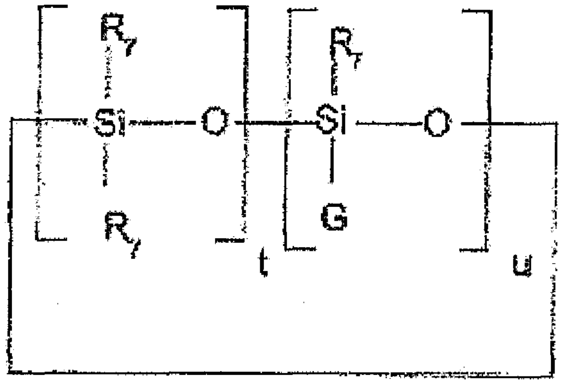

(6)

wobei:

- die Variablen $R_7$, die gleich oder verschieden sein können, aus $C_1$-$C_{10}$-Alkyl-, Phenyl-, 3,3,3-Trifluorpropyl- und Trimethylsiloxyresten ausgewählt sind, wobei zahlenmäßig mindestens 80 % der Reste $R_7$ Methyl sind,
- die Variablen D, die gleich oder verschieden sein können, aus den Resten $R_7$ und dem Rest G ausgewählt sind,
- r für eine ganze Zahl zwischen 0 und 50 inklusive steht und s für eine ganze Zahl zwischen 0 und 20 inklusive ist und dann, wenn s = 0, mindestens eines der beiden Symbole D für G steht,
- u für eine ganze Zahl zwischen 1 und 6 inklusive steht und t für eine ganze Zahl zwischen 0 und 10 inklusive steht, wobei es sich versteht, dass t + u gleich oder größer als 3 ist,
- und das Symbol G der obigen Formel (2) entspricht.

**10.** Zusammensetzung nach Anspruch 9, wobei die Benzotriazol-UV-Filtersubstanz aus denjenigen der Formel (5) ausgewählt ist, insbesondere aus denjenigen, für die die Reste D beide für Reste $R_7$ stehen.

**11.** Zusammensetzung nach Anspruch 9 oder 10, wobei die Benzotriazol-UV-Filtersubstanz der Formel (5) mindestens eine und noch weiter bevorzugt alle der folgenden Eigenschaften aufweist:

- D steht für einen Rest $R_7$,
- $R_7$ steht für Alkyl und noch weiter bevorzugt für Methyl,
- r liegt zwischen 0 und 15 inklusive, s liegt zwischen 1 und 10 inklusive,
- n ist nicht gleich null und ist vorzugsweise gleich 1, und Y ist dann aus Methyl, tert-Butyl und $C_1$-$C_4$-Alkoxy ausgewählt,
- Z steht für Wasserstoff oder Methyl,
- m = 0 oder [m = 1 und X = O],
- p ist gleich 1.

**12.** Zusammensetzung nach einem der Ansprüche 9 bis 11, wobei die lipophile Benzotriazol-UV-Filtersubstanz der

nachstehenden allgemeinen Formel (7) entspricht:

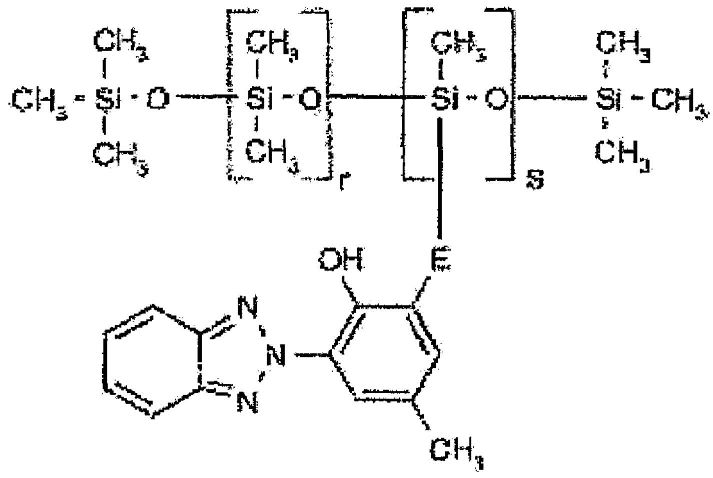

(7)

wobei $0 \leq r \leq 10$,
$1 \leq s \leq 10$,
und wobei E für den zweiwertigen Rest:

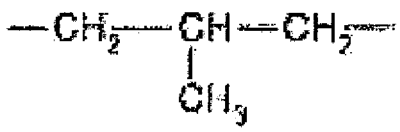

steht.

**13.** Zusammensetzung nach Anspruch 12, wobei es sich bei der Benzotriazol-UV-Filtersubstanz der Formel (7) um die Verbindung handelt, die der folgenden Formel entspricht:

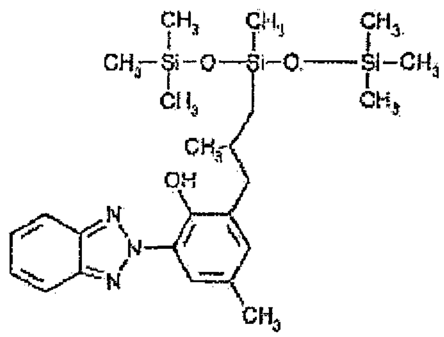

Verbindung (a)

**14.** Zusammensetzung nach einem der Ansprüche 1 bis 6, wobei die Bisresorcinyltriazin-Verbindung der nachstehenden Formel (II) entspricht:

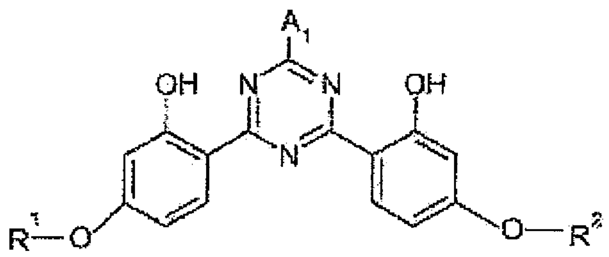

(II)

wobei:

(i) die Reste $R_1$ und $R_2$, die gleich oder verschieden sein können, für einen $C_3$-$C_{18}$-Alkylrest, einen $C_2$-$C_{18}$-Alkenylrest oder einen Rest der Formel $-CH_2$-CH (OH) $-CH_2$-$OT_1$, wobei $T_1$ für ein Wasserstoffatom oder einen $C_1$-$C_8$-Alkylrest steht, stehen; oder
(ii) die Reste $R_1$ und $R_2$, die gleich oder verschieden sein können, für einen Rest der nachstehenden Formel (III) stehen:

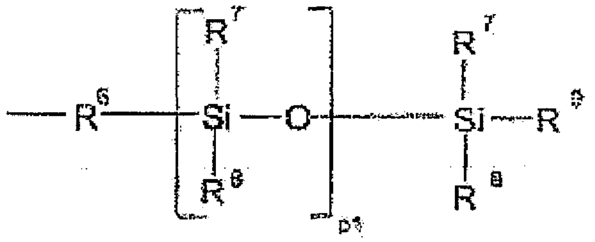

(III)

wobei:

- $R_6$ für eine kovalente Bindung; einen linearen oder verzweigten $C_1$-$C_4$-Alkylenrest oder einen Rest der Formel $C_{m1}H_{2m1}$- oder $-C_{m1}H_{2m1}$-O-, wobei $m_1$ für eine Zahl von 1 bis 4 steht, steht;
- $p_1$ für eine Zahl von 0 bis 5 steht;
- die Reste $R_7$, $R_8$ und $R_9$, die gleich oder verschieden sein können, für einen $C_1$-$C_{18}$-Alkylrest, einen $C_1$-$C_{18}$-Alkoxyrest oder einen Rest der Formel:

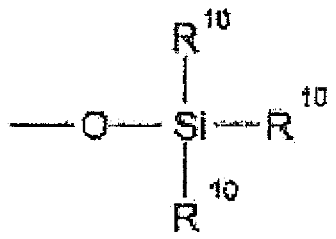

(IV)

wobei $R_{10}$ für einen $C_1$-$C_5$-Alkylrest steht, stehen:

- $A_1$ für einen Rest gemäß einer der folgenden Formeln steht:

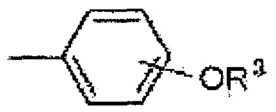 (V)

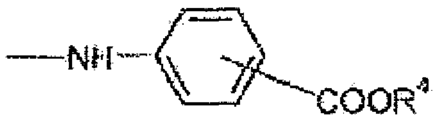 (VI)

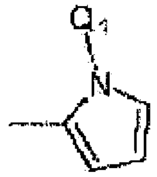 (VII)

wobei:

- $R_3$ für ein Wasserstoffatom, einen $C_1$-$C_{10}$-Alkylrest oder einen Rest der Formel -$(CH_2CHR^5\text{-}O)_{n1}R^4$, wobei $n_1$ für eine Zahl von 1 bis 16 steht, steht,
- $R^4$ für Wasserstoff, ein Metallkation M, einen $C_1$-$C_5$-Alkylrest oder einen Rest der Formel -$(CH_2)m_2$-$OT_1$, wobei $m_2$ für eine Zahl von 1 bis 4 steht und $T_1$ die in Absatz (i) angegebene Bedeutung besitzt, steht,
- $R^5$ für Wasserstoff oder Methyl öder einen Rest der Struktur -$CH_2$-CH- (OH) -$CH_2OT_1$, wobei $T_1$ die in Absatz (i) angegebene Bedeutung besitzt, steht,
- $Q_1$ für einen $C_1$-$C_{18}$-Alkyrest steht.

**15.** Zusammensetzung nach Anspruch 14, wobei die Bisresorcinyltriazin-Verbindung der Formel (II) aus

- 2,4-Bis{[4-(3-(2-propyloxy)-2-hydroxypropyloxy)-2-hydroxy]phenyl)-6-(4-methoxyphenyl)-1,3,5-triazin;
- 2,4-Bis{[4-(2-ethylhexyloxy)-2-hydroxy]phenyl}-6-[4-(2-methoxyethylcarboxyl)phenylamino]-1,3,5-triazin;
- 2,4-Bis{[4-tris(trimethylsiloxysilylpropyloxy)-2-hydroxy]phenyl}-6-(4-methoxyphenyl)-1,3,5-triazin;
- 2,4-Bis{[4-(2''-methylpropenyloxy)-2-hydroxy]-phenyl}-6-(4-methoxyphenyl)-1,3,5-triazin;
- 2,4-Bis{[4-(1',1',1',3',5',5',5'-heptamethyltrisiloxy-2''-methylpropyloxy)-2-hydroxylphenyl}-6-(4-methoxyphenyl)-1,3,5-triazin;
- 2,4-Bis{[4-(3-(2-propyloxy)-2-hydroxypropyl-oxyl-2-hydroxy]phenyl)-6-[(4-ethylcarboxyl)-phenylamino]-1,3,5-triazin;
- 2,4-Bis([4-(2-ethylhexyloxy)-2-hydroxy]phenyl}-6-(1-methylpyrrol-2-yl)-1,3,5-triazin

und Mischungen davon ausgewählt ist und es sich noch spezieller bei der Bisresorcinyltriazin-Verbindung der Formel (II) um 2,4-Bis{[4-(2-ethyl-hexyloxy)-2-hydroxy]phenyl)-6-(4-methoxyphenyl)-1,3,5-triazin handelt.

**16.** Zusammensetzung gemäß einem der vorhergehenden Ansprüche zur Verwendung bei einem Verfahren zum Pflegen und/oder Schminken eines Keratinmaterials, bei dem man die Zusammensetzung auf die Oberfläche des Keratinmaterials aufbringt.

**17.** Zusammensetzung gemäß einem der vorhergehenden Ansprüche zur Verwendung bei einem Verfahren zum Einschränken des Dunkelwerdens der Haut und/oder zum Verbessern der Farbe und/oder Einheitlichkeit des Teints, bei dem man die Zusammensetzung auf die Oberfläche der Haut aufbringt.

**18.** Zusammensetzung gemäß einem der vorhergehenden Ansprüche zur Verwendung bei einem Verfahren zum Verhindern und/oder Behandeln der Anzeichen von Alterung eines Keratinmaterials, bei dem man die Zusammensetzung auf die Oberfläche des Keratinmaterials aufbringt.

**Revendications**

1. Composition cosmétique ou dermatologique comprenant dans un support physiologiquement acceptable :

   a) au moins une phase huileuse et
   b) au moins un composé mérocyanine répondant à la formule (I) suivante ainsi que ses formes géométriques isomères E/E- ou E/Z-:

   (I) 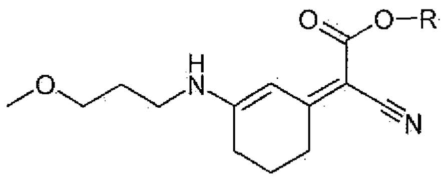

   dans laquelle
   R est un groupement alkyle en $C_1$-$C_{22}$, un groupement alcényle en $C_2$-$C_{22}$, un groupement alcinyle en $C_2$-$C_{22}$, un groupement cycloalkyle en $C_3$-$C_{22}$ ou un groupement cycloalcényle en $C_3$-$C_{22}$, lesdits groupements pouvant être substitués par un ou plusieurs O et
   c) au moins un filtre UV benzotriazole lipophile et/ou un composé bis-résorcinyl triazine ;
   ladite composition contenant moins de 2% de cyclohexasiloxane par rapport au poids total de la composition quand elle contient au moins un filtre uv benzotriazole lipophile, à l'exclusion de l'émulsion H/E suivante dans laquelle les quantités des composants sont exprimées en pourcentage en poids par rapport au poids total de la composition :

| Composants | % en poids |
|---|---|
| Caprylylglycol | 0,5 |
| Copolymère de triglycéride caprylique/caprique et d'acrylates de sodium (Luvigel EM-BASF) | 3 |
| Triéthanolamine | 0,2 |
| Salicylate d'éthylhexyle | 5 |
| Drométrizole-trisiloxane | 2 |
| Laurylcarbamate d'inuline (Inutec SP1-ORAFTI) | 0,3 |
| Cyclohexasiloxane | 2 |
| Glycérine | 5 |
| Propylène glycol | 10 |
| Benzoate d'alkyle en $C_{12}$-$C_{15}$ (Finsolv TN-INNOSPEC ACTIVE CHEMICAL) | 7 |
| Octocrylène | 7 |
| Butylméthoxydibenzoylméthane | 3 |
| (2Z)-Cyano{3-[(3-méthoxypropyl) amino] cyclohex-2-en-1-ylidène}éthanoate d'éthyle | 3 |
| Acide téréphtalylidène dicamphorsulfonique | 0,5 |
| Conservateur | 0,8 |
| EDTA disodique | 0,1 |
| Eau | q.s. 100 |

2. Composition selon la revendication 1, où le ou les composés mérocyanines de formule (I) sont choisis parmi ceux où R est un alkyle en $C_1$-$C_{22}$, pouvant être substitué par un ou plusieurs O.

3. Composition selon la revendication 1 ou 2, où le ou les composés mérocyanines de formule (I) sont choisis parmi

les composés suivants ainsi que leurs formes géométriques isomères E/E- ou E/Z- :

| | | | |
|---|---|---|---|
| 1 | 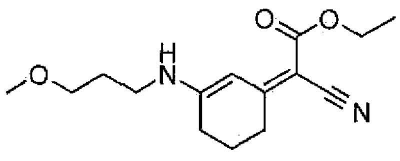<br>ethyl (2Z)-cyano{3-[(3-methoxypropyl) aminolcyclohex-2-en-1-ylidene}ethanoate | 4 | 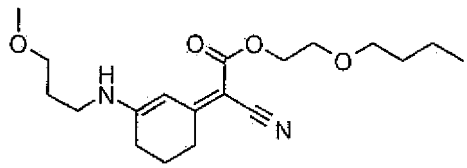<br>2-butoxyethyl(2Z)-cyano{3-[(3-methoxypropyl} amino]cyclohex-2-en-1-ylidene}ethanoate |
| 2 | 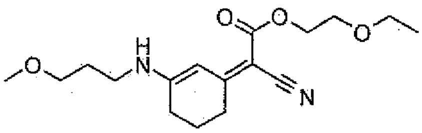<br>2-ethoxyethyl (2Z)-cyano{3-[(3-methoxypropyl) amino]cyclohex-2-en-1-ylidene}ethanoate | 5 | 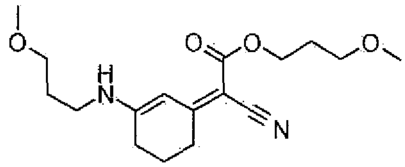<br>3-methoxypropyl (2Z) -cyano{3-[(3-methoxypropyl) amino] cyclohex-2-en-1-ylidene}ethanoate |
| 3 | 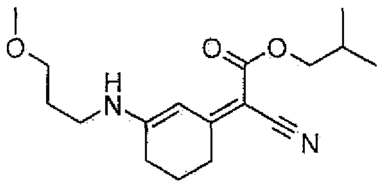<br>2-methylpropyl(2Z)-cyano{3-[(3-methoxypropyl) amino]cyclohex-2-en-1-ylidene}ethanoate | 6 | 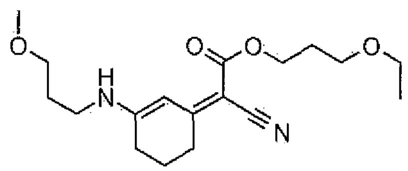<br>3-ethoxypropyl (2z)-cyano{3-[(3-methoxypropyl) amino]cyclohex-2-en-1-ylidene}ethanoate |

**4.** Composition selon la revendication 3, où le ou les composés mérocyanines de formule (I) sont choisis parmi les composés suivants ainsi que leurs formes géométriques isomères E/E- ou E/Z- :

**5.** Composition selon la revendication 4, où le composé mérocyanine est le 2-éthoxyéthyle (2z)-cyano{3-[(3-méthoxypropyl)amino]cyclohex-2-èn-1-ylidène}éthanoate (2) dans sa configuration géométrique E/Z de structure suivante :

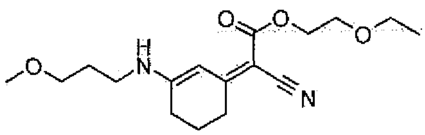

et/ou dans sa configuration géométrique E/E de structure suivante :

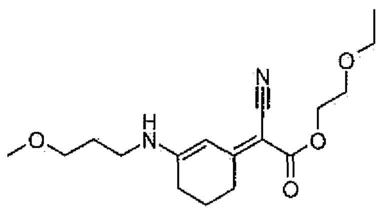

**6.** Composition selon l'une quelconque des revendications 1 à 5, où le ou les composés mérocyanines de formule (I) sont présents dans une concentration allant de 0,1% à 10% en poids, et préférentiellement de 0,2% à 5% en poids par rapport au poids total de la composition.

**7.** Composition selon l'une quelconque des revendications 1 à 6, où le filtre UV benzotriazole lipophile est choisi parmi :

- le 2-benzotriazolyl-4-tert-octylphénol,
- le 2-(2H-benzotriazol-2-yl)-4-methylphénol,
- le 2-2(H-benzotriazole-2-yl)-p-crésol,
- le 2-(2'-hydroxy-3'-butyl-5'-méthylphényl)benzotriazole,
- le 2-(2'-hydroxy-5'-t-octylphényl)benzotriazole,
- le benzotriazolyl dodécyl p-crésol,
- le 2-t-butyl-6-(5-chloro-2H-benzotriazol-2-yl)-p-crésol,
- les composés benzotriazoles de formule

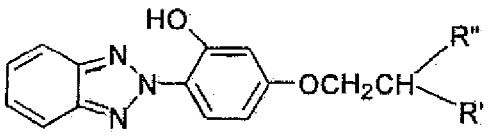

dans laquelle R' désigne un alkyle linéaire en $C_1$-$C_6$ et R'' un alkyle en $C_1$-$C_3$ en particulier le 2-[4-(2-éthylhexyloxy)-2hydroxyphényl]-2H-benzotriazole et 2-(2-hydroxy-4-isobuthoxyphényl)-2H-benzotriazole
- et leurs mélanges.

**8.** Composition selon l'une quelconque des revendications 1 à 6, où le filtre UV benzotriazole lipophile est choisi parmi les silanes ou les siloxanes à fonction benzotriazole comprenant au moins une unité de formule (1) suivante :

$$O_{(3-a)/2}Si(R_7)_a\text{- } G \qquad (1)$$

dans laquelle :

- $R_7$ représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante :

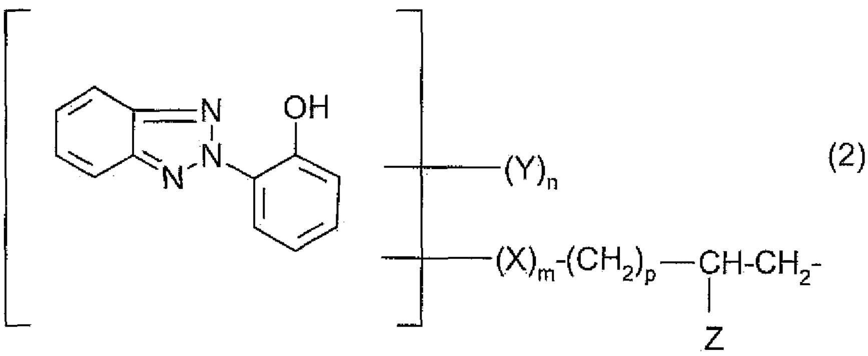

(2)

dans laquelle :

- Y, identiques ou différents, sont choisis parmi les radicaux alkyle en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidènedioxy dans lequel le groupe alkylidène contient de 1 à 2 atomes de carbone,
  - X représente O ou NH,
  - Z représente l'hydrogène ou un radical alkyle en $C_1$-$C_4$,
  - n est un nombre entier compris entre 0 et 3 inclusivement,
  - m est 0 ou 1,
  - p représente un nombre entier compris entre 1 et 10 inclusivement.

**9.** Composition selon la revendication 8, où le filtre UV benzotriazole lipophile répond à la formule (5) ou (6) suivante :

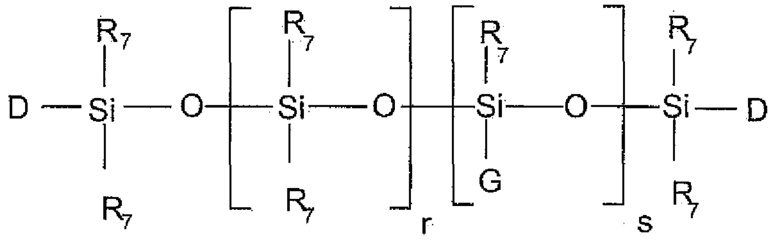

(5)

ou

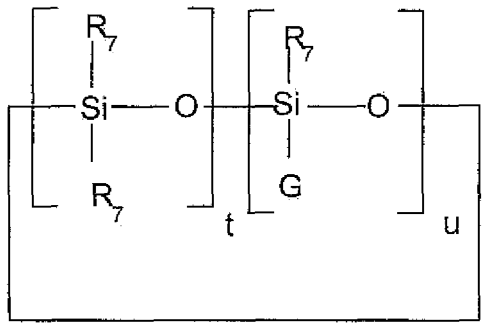

(6)

dans lesquelles :

- $R_7$, identiques ou différents, sont choisis parmi les radicaux alkyle en $C_1$-$C_{10}$, phényle, trifluoro-3,3,3-propyle et triméthylsilyloxy, au moins 80% en nombre des radicaux $R_7$ étant méthyle,
- D, identiques ou différents, sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

**10.** Composition selon la revendication 9, où le filtre UV benzotriazole est choisi parmi ceux de formule (5), en particulier parmi ceux pour lesquels les radicaux D sont tous les deux des radicaux $R_7$.

**11.** Composition selon la revendication 9 ou 10, où le filtre UV benzotriazole de formule (5) présente au moins l'une, et encore plus préférentiellement l'ensemble des caractéristiques suivantes :

- D est un radical $R_7$
- $R_7$ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert-butyle ou alcoxy en $C_1$-$C_4$,
- Z est hydrogène ou méthyle,
- m=0, ou [m=1 et X=O]
- p est égal à 1.

**12.** Composition selon l'une quelconque des revendications 9 à 11, où le filtre UV benzotriazole lipophile répond à la formule générale (7) suivante :

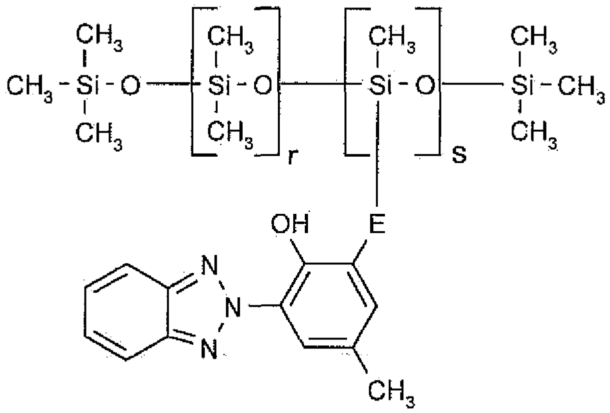

(7)

avec $0 \leq r \leq 10$,
$1 \leq s \leq 10$,
et où E représente le radical divalent :

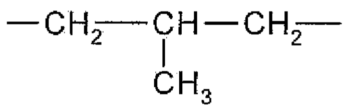

**13.** Composition selon la revendication 12, où le filtre UV benzotriazole de formule (7) est le composé répondant à la formule suivante :

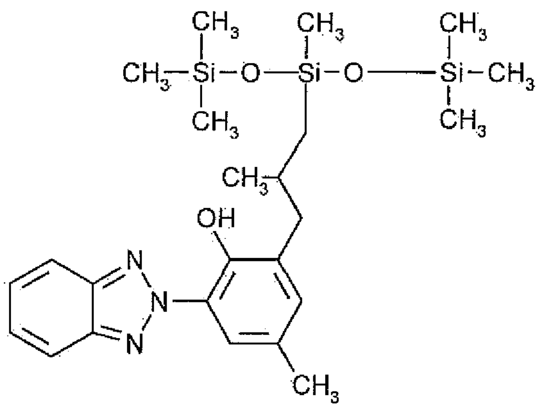

composé (a)

**14.** Composition selon l'une quelconque des revendications 1 à 6, où le composé bis-résorcinyl triazine répond à la formule (II) suivante :

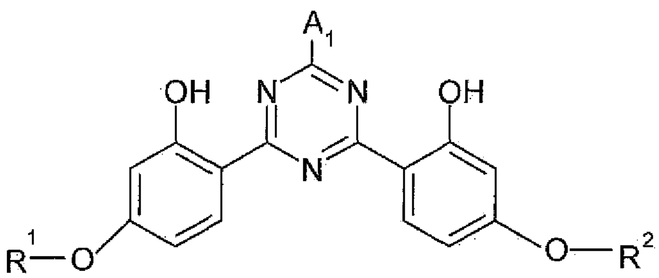

(II)

dans laquelle :

(i) les radicaux $R^1$ et $R^2$, identiques ou différents, désignent un radical alkyle en $C_3$-$C_{18}$ ; un radical alcényle en $C_2$-$C_{18}$ ou bien un reste de formule $-CH_2-CH(OH)-CH-OT_1$ où $T_1$ est un atome d'hydrogène ou un radical

alkyle en $C_1$-$C_8$ ; ou bien

(ii) les radicaux $R^1$ et $R^2$, identiques ou différents, désignent un reste de formule (III) suivante :

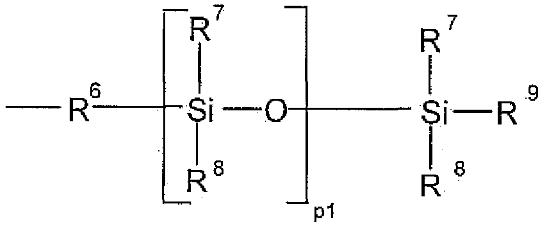

(III)

dans laquelle :

- $R^6$ désigne une liaison covalente ; un radical alkylène linéaire ou ramifié en $C_1$-$C_4$ ou bien un reste de formule $-C_{m1}H-_{2m1}-$ ou $-C_{m1}H_{2m1}-O-$ où m1 est un nombre de 1 à 4 ;
- p1 est un nombre de 0 à 5 ;
- les radicaux $R^7$, $R^8$ et $R^9$, identiques ou différents, désignent un radical alkyle en $C_1$-$C_{18}$ ; un radical alcoxy en $C_1$-$C_{18}$ ou un reste de formule :

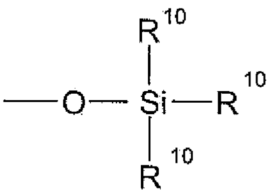

(IV)

où $R^{10}$ est un radical alkyle en $C_1$-$C_5$ ;

- $A_1$ désigne un reste répondant à l'une des formules suivantes :

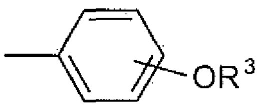

(V)

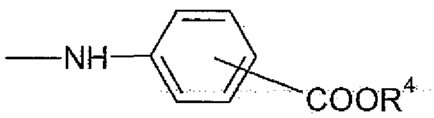

5
(VI)

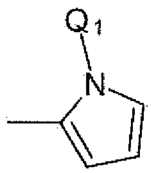

(VII)

dans lesquelles :

- $R^3$ désigne un atome d'hydrogène, un radical alkyle en $C_1$-$C_{10}$, un radical de formule :
- $(CH_2CHR^5-O)_{n1}R^4$ où n1 est un nombre de 1 à 16,
- $R^4$ désigne hydrogène, un cation métallique M, un radical alkyle en $C_1$-$C_5$ ou un reste de formule
- $\{CH_2)_{m2}-OT_1$ où $m_2$ est un nombre de 1 à 4 et $T_1$ a la même signification indiquée dans le paragraphe (i),
- $R^5$ est hydrogène ou méthyle ou bien un reste de structure $-CH_2-CH-$ $(OH)-CH_2OT_1$ avec $T_1$ ayant la même signification indiquée dans le paragraphe (i) ,

- $Q_1$ est un radical alkyle en $C_1$-$C_{18}$.

**15.** Composition selon la revendication 14, où le composé bis-résorcinyl triazine de formule (II) est choisi parmi :

- la 2, 4-bis {[4-(3-(2-propyloxy)-2-hydroxypropyloxy)-2-hydroxy]-phényl}-6-(4-méthoxyphényl)-1,3,5-triazine ;
- la 2,4-bis{[4-(2-éthylhexyloxy)-2-hydroxy]-phényl}-6-[4-(2-méthoxyéthyl-carboxyl)phénylamino]-1,3,5-triazine ;
- la 2,4-bis{[4-tris(triméthylsiloxysilylpropyloxy)-2-hydroxy]-phényl}-6-(4-méthoxyphényl)-1,3,5-triazine ;
- la 2,4-bis{[4-(2''-méthylpropenyloxy)-2-hydroxy]phényl}-6-(4-méthoxyphényl)-1,3,5-triazine ;
- la 2,4-bis{[4-(1',1',1',3',5',5',5'-heptaméthyltrisiloxy-2''-méthylpropyloxy)-2-hydroxy] phényl}-6-(4-méthoxyphényl)-1,3,5-triazine.
- la 2,4-bis{[4-(3-(2-propyloxy)-2-hydroxypropyloxy]-2-hydroxy]-phényl}-6-[(4-éthylcarboxyl) phénylamino]-1,3,5-triazine ;
- la 2,4-bis{[4-(2-éthylhexyloxy)-2-hydroxylphényl}-6-(1-méthylpyrrol-2-yl)-1,3,5-triazine et leurs mélanges, et encore plus particulièrement le composé bis-résorcinyl triazine de formule (II) est la 2,4-bis {[4-(2-éthyl-hexyloxy)-2-hydroxyl-phényl)-6-(4-méthoxyphényl)-1,3,5-triazine.

**16.** Composition telle que définie dans l'une quelconque des revendications précédentes pour utilisation dans un procédé de soin et/ou de maquillage d'une matière kératinique comprenant l'application sur la surface de ladite matière kératinique de ladite composition.

**17.** Composition telle que définie dans l'une quelconque des revendications précédentes pour utilisation dans un procédé pour limiter l'assombrissement de la peau et/ou améliorer la couleur et/ou l'homogénéité du teint comprenant l'application sur la surface de la peau de ladite composition.

**18.** Composition telle que définie dans l'une quelconque des revendications précédentes pour utilisation dans un procédé pour prévenir et/ou traiter les signes du vieillissement d'une matière kératinique comprenant l'application sur la surface de la matière kératinique de ladite composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2326405 A
- FR 2440933 A
- EP 0114607 A
- WO 0025370 A
- US 5869030 A
- EP 1977730 A, Shiseido
- EP 0660701 A
- EP 0775698 A
- US 4195999 A
- WO 2004006878 A
- WO 2008090066 A
- WO 2011113718 A
- WO 2009027258 A
- WO 2013010590 A
- WO 2013011094 A
- WO 2013011480 A
- WO 2007071582 A
- US 4749643 A
- EP 0392883 A
- EP 0708108 A
- EP 0711778 A
- EP 711779 A
- US 3220972 A
- US 3697473 A
- US 4340709 A
- US 4316033 A
- US 4328346 A
- EP 0742003 A
- WO 2007068371 A
- WO 2008155059 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 2007071584 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0841341 A
- EP 0518773 A
- WO 9206778 A
- FR 2315991
- FR 2416008
- US 4077441 A
- US 4850517 A


### Non-patent literature cited in the description


- *IP COM JOURNAL,* 23 February 2009
- *IP COM JOURNAL,* 12 November 2009
- *IP COM Journal,* 03 April 2004
- *IP.com Journal,* 2009, vol. 9 (5A), 29-30
- *CHEMICAL ABSTRACTS,* 919803-06-8
- Symmetrical Triazine Derivatives. IP.COM IPCOM000031257 Journal. INC West Henrietta, 20 September 2004
- **BANGHAM ; STANDISH ; WATKINS.** *J. Mol. Biol.,* 1965, vol. 13, 238
- **B.L. DIFFEY.** *J. Soc. Cosmet. Chem.,* 1989, vol. 40, 127-133